# EUROPÄISCHE PATENTSCHRIFT

(11) **EP 4 321 084 B1**
(45) Veröffentlichungstag und Bekanntmachung des Hinweises auf die Patenterteilung: **02.09.2026**
(21) Anmeldenummer: 23183360.9
(22) Anmeldetag: 04.07.2023
(51) Int. Cl.: A61B 3/10, G06T 13/00, G06T 15/06

(54) **SYSTEM ZUM ERFASSEN UND VISUALISIEREN VON OCT-SIGNALEN**
SYSTEM FOR RECORDING AND VISUALIZING OCT SIGNALS
SYSTÈME DE DÉTECTION ET DE VISUALISATION DE SIGNAUX OCT

(30) Priorität: 10.08.2022 DE 102022120203
(43) Veröffentlichungstag der Anmeldung: 14.02.2024
(73) Patentinhaber: Carl Zeiss Meditec AG, 07745 Jena (DE)
(72) Erfinder: HAUGER, Christoph, 73431 Aalen (DE); SCHMOLL, Tilman, 10902 Wien (AT); HECKER-DENSCHLAG, Nancy, 89077 Ulm (DE); STEFFEN, Joachim, 73463 Westhausen (DE)
(74) Vertreter: Gulde & Partner

(56) Entgegenhaltungen:
- US-A1- 2012 092 615
- US-A1- 2021 169 320
- US-A1- 2021 169 324
- US-A1- 2022 117 696

## Beschreibung

### Gegenstand der Erfindung

Die vorliegende Erfindung betrifft ein System zum Erfassen und Visualisieren von OCT-Signalen, insbesondere zum Erfassen von zeitaufgelösten OCT-Signalen und zum Visualisieren der OCT-Signale in Form von zeitaufgelösten OCT-Bildern. Die vorliegende Erfindung betrifft ferner ein Verfahren zum Erfassen und Visualisieren von OCT-Signalen.

### Technologischer Hintergrund

Der Einsatz von technologischen Hilfsmitteln ist fester Bestandteil der modernen Medizin. Sowohl bildgebende Verfahren als auch robotische Systeme werden mittlerweile in der Chirurgie ebenso selbstverständlich eingesetzt wie in der Diagnostik. Das Verwenden bildgebender Verfahren ermöglicht dabei die Darstellung sowie Diskriminierung vielfältiger Strukturen im Patienten und die vom Patienten gewonnenen Bilddaten können vorteilhaft in der Diagnostik, als auch in therapeutischen und chirurgischen Verfahren eingesetzt werden.

Beispielsweise kann ein Operateur anhand von Bilddaten eines Patienten einen chirurgischen Eingriff besser planen und sich auch bei der Durchführung des Eingriffs unterstützen lassen. Für die Unterstützung von Operateuren bei der Durchführung chirurgischer Eingriffe kommen robotische Visualisierungssysteme zum Einsatz. Diese weisen in der Regel zumindest eine Kamera zur Aufnahme von Bildern des zu operierenden Bereichs auf, die von einem Stativ mit einer Gelenkstruktur getragen wird. Das Stativ erlaubt es, die Kamera durch Translations- und/oder Rotationsbewegungen relativ zum Subjekt zu positionieren, um Bilder eines gewünschten Sichtfelds (*field of view - FOV*) des zu operierenden Bereichs zu erfassen. Die Verwendung optischer Stereokameras ermöglicht dabei das Erfassen von 3D Bilddaten.

Neben der Erfassung von Oberflächeninformationen eines gewünschten Sichtfelds, beispielsweise anhand von reflektiertem beziehungsweise rückgestreutem sichtbaren Licht, existieren mittlerweile auch Methoden zum Erfassen von Tiefeninformationen des Sichtfelds. Diese Methoden umfassen die optische Kohärenztomographie (*optical coherence tomography, OCT*), welche die dreidimensionale mikroskopische Abbildung von optisch transparenten und/oder reflektierenden Objekten und somit die Aufnahme von Volumenbildern des biologischen Gewebes im betrachteten Sichtfeld erlaubt. Bei der optischen Kohärenztomographie (OCT) handelt es sich im Wesentlichen um eine interferometrische Methode unter Verwendung von breitbandigem Licht mit geringer Kohärenzlänge. Systeme zum Erfassen von OCT Daten weisen daher ein Interferometer und eine breitbandige Lichtquelle mit einer spektralen Breite von mehr als 1% der zentralen Wellenlänge auf.

Die Erfassung von OCT Daten kann sequentiell oder parallel erfolgen. Die sequentielle Erfassung von OCT Daten erfolgt beispielsweise indem ein kohärenzarmer Quelllichtstrahl an einem Strahlteiler in einen Probenstrahl und in einen Referenzstrahl geteilt wird, die durch zwei Arme eines Interferometers geschickt werden, wobei im Referenzstrahlengang ein beweglicher Referenzspiegel und im Objektstrahlengang das zu untersuchende Objekt angeordnet ist. Durch Verschiebung des Referenzspiegels kann ein Gangunterschied zwischen Objekt- und Referenzstrahl und somit die vermessene Tiefe eingestellt werden. Mittels eines Spiegels im Objektstrahlengang wird der Objektstrahl zweidimensional über die Probe gerastert, was im Ergebnis eine dreidimensionale Abtastung der Probe ermöglicht.

Bei solch einer Erfassung von OCT Daten in der Zeitdomäne (*time domain OCT - TD OCT*) korrespondiert die spektrale Breite der Lichtquelle Δλ zu einer Kohärenzlänge L_{C} von L_{C}=λ*/Δλ. Die axiale Auflösung eine OCT-System korrespondiert zur Kohärenzlänge L_{C} des eingesetzten Lichts und bezeichnet das Auflösungsvermögen von Objekten, die entlang der optischen Achse einen Abstand von zumindest der Kohärenzlänge aufweisen. Beispielsweise hat eine Lichtquelle im Nahinfrarotbereich mit einer zentralen Wellenlänge von 800 nm und einer spektralen Breite von 80 nm eine Kohärenzlänge von 7 µm und ein OCT System mit einer solchen Quelle hat somit eine axiale Auflösung von etwa 1-10 µm. Die transversale Auflösung eines OCT Systems ist durch die im Objektstrahlengang verwendete Optik bestimmt, insbesondere durch die das Licht auf das zu untersuchende Objekt fokussierende Objektlinse.

Eine sequentielle Erfassung von OCT Daten ist auch in der Frequenzdomäne möglich (*frequency domain OCT - FD OCT*), wobei in der Regel zwischen der Verwendung einer durchstimmbaren Quelle (*swept source OCT*) und der Verwendung eines dispersiven Detektors (*spectral domain OCT- SD OCT*) unterschieden wird. Bei der *swept source OCT* wird die Frequenz der Anregungslichtquelle, häufig ein Laser, durchgestimmt, wodurch ein Gangunterschied zwischen Proben- und Referenzstrahl und somit die abgetastete Probentiefe auch ohne verschiebbaren Referenzspiegel variiert werden kann. Bei der SD *OCT* wird ebenfalls eine breitbandige Lichtquelle verwendet, jedoch werden die Frequenzkomponenten des Interferenzsignals vor der Detektion separiert, beispielsweise durch ein optisches Gitter.

Mittels OCT sind Schnitt- und Volumendaten von biologischem Gewebe erfassbar und kann der Informationsgehalt für einen Operateur deutlich erhöht werden. Somit ist eine Integration von OCT in Operationsmikroskope wünschenswert, um sowohl Videodaten der Oberfläche eines gewünschten Sichtfelds als auch Tiefen- und/oder Schnittbilder des Sichtfelds zu darstellen zu können. Sofern Operationssysteme bislang dazu in der Lage sind volumetrische OCT-Bilder zu liefern, ist deren Aufnahmezeit relativ hoch und ist zudem das Rendern aus Zeit- und Ressourcengründen allein auf die Nachbearbeitung beschränkt. Eine Volumenbildgebung in Echtzeit während der Operation war daher bislang nicht möglich.

Mit weiteren Fortschritten in der OCT-Technologie sowie der Kapazität und Geschwindigkeit von Prozessoren zur Grafikverarbeitung (GPUs) werden jedoch schnellere OCT-Verfahren zur intraoperativen Bildgebung verfügbar. Die dadurch mögliche Darstellung von OCT-Volumenbildern in Echtzeit ermöglicht jedoch vielfältige Möglichkeiten zur Darstellung der erfassten OCT-Signale, wobei Verfahren hierfür noch nicht zur Verfügung stehen. Insbesondere fehlen Verfahren, die eine an medizinische beziehungsweise chirurgische Anwendungen optimierte Form der Darstellung der OCT-Signale ermöglichen. Die Verwendung nicht optimaler Verfahren birgt die Gefahr der Überforderung beziehungsweise Ablenkung des Nutzers, was gerade bei Operateuren schwerwiegende Folgen haben kann.

US 2021/169320 A1 und US 2021/169324 A1 offenbaren Systeme zum Begleiten ophthalmologischer Eingriffe mit einer integrierten stereoskopischen Visualisierungskamera und einem OCT-System, wobei Daten verschiedener Bildgebungsmodi fusioniert werden sollen, um eine synthetisierte Darstellung eines Objekts für einen Nutzer bereitzustellen, wobei diese Darstellung Schrägdarstellungen umfasst.

US 2022/117696 A1 offenbart ein operationsmikroskopisches System, das eine Registrierung von zweidimensionalen Videobilddaten und dreidimensionalen OCT Daten ermöglichen soll.

Die US 2012/092615 A1 beschreibt ein Operationsmikroskop mit zwei Mikroskop-Bildgebungspfaden und einer OCT-Einheit mit einem in zumindest einem der Mikroskop-Bildgebungspfade angeordneten Strahlteiler, der einen Teil des von der Probe (Patientenauge) kommenden Lichts zu einem Mikroskop-Bildgebungspfad und einen anderen Teil desselben Lichts zu der OCT-Einheit leitet.

Die Aufgabe der vorliegenden Erfindung ist es, die Nachteile des Standes der Technik zu überwinden oder zumindest zu verringern und ein verbessertes System und ein verbessertes Verfahren zum Erfassen und Visualisieren von OCT-Signalen bereitzustellen.

### Beschreibung der Erfindung

Die erfindungsgemäße Aufgabe wird gelöst durch die Gegenstände der unabhängigen Patentansprüche. Bevorzugte Weiterbildungen sind Gegenstand der Unteransprüche.

Ein erster Aspekt der vorliegenden Offenbarung betrifft ein System zum Erfassen und zum Visualisieren von OCT-Signalen, insbesondere zum Erfassen und Visualisieren solcher Signale mittels eines medizintechnischen Geräts, beispielsweise eines Operationsmikroskops.

Das System gemäß der vorliegenden Offenbarung weist ein OCT-System auf. Das OCT System weist bevorzugt eine zum Beleuchten einer Probe ausgebildete breitbandige Lichtquelle auf. Bei dieser Lichtquelle handelt es sich bevorzugt um einen durchstimmbaren Laser (*swept source*), beispielsweise ein Breitband-Laser, ein Superkontinuum-Laser und/oder ein Ultrakurzpulslaser. Dabei kann ein durchstimmbarer Laser zu einem gegebenen Zeitpunkt eine schmalbandige Lichtquelle sein, deren Mittelfrequenz jedoch zeitlich gezielt variierbar ist oder aus einer Mehrzahl schmalbandiger Lichtquellen gebildet sein. Es können jedoch auch andere breitbandige Quellen zum Einsatz kommen, beispielsweise eine Superlumineszenzdiode, beispielsweise in einer *FD-OCT.*

Das OCT-System weist ferner bevorzugt ein zum Erzeugen und Überlagern eines Probenstrahls und eines Referenzstrahls ausgebildetes Interferometer auf, beispielsweise ein Michelson, Mach-Zehner oder Koster Interferometer. Das Interferometer weist bevorzugt einen Strahlteiler zum Erzeugen und Überlagern von Proben- und Referenzstrahl aus dem Licht der breitbandigen Quelle, einen Referenzstrahlengang und einen Probenstrahlengang auf. Ferner bevorzugt weist das Interferometer Mittel zum Einstellen einer untersuchten Probentiefe auf. Dabei kann es sich je nach Messmethode um ein Mittel zum Erzeugen eines Gangunterschieds (wie eines im Referenzstrahl verschiebbaren Spiegels bei einer SD-OCT), ein Mittel zum Separieren von Licht eines bestimmten Gangunterschieds (wie ein optisches Gitter bei einer FD-OCT) oder um Mittel zum Erzeugen von Licht eines bestimmten Gangunterschieds (wie eine durchstimmbaren Quelle bei einer *swept source*-OCT) handeln.

Das OCT-System weist ferner einen zum Abrastern der Probe mit dem Probenstrahl ausgebildeten Scanmechanismus auf. Der Scanmechanismus ist insbesondere dazu ausgebildet, den Probenstrahl in zwei Dimensionen über die Probe zu rastern. Bevorzugt handelt es sich bei dem Scanmechanismus um einen Scanspiegel, es können jedoch auch andere Scanmechanismen, wie beispielsweise ein Gasfaserscanner, ein Prismenscanner, ein Palmer-Scanner oder dergleichen zum Einsatz kommen. Bei einem für Full-Field-OCT eingerichteten OCT-System ist ein Scanmechanismus verzichtbar.

Das OCT-System weist ferner einen zum Erfassen eines durch Überlagerung von Probenstrahl und Referenzstrahl erzeugten Interferenzmusters ausgebildeten Detektor auf. Bei dem Detektor handelt es sich beispielsweise um einen Liniendetektor, ein zweidimensionales Detektorarray, einen Photodetektor beziehungsweise einen dispersiven Detektor. Der Detektor ist beispielsweise als CCD oder als ein CMOS Detektor ausgebildet.

Das System gemäß der vorliegenden Offenbarung weist ferner ein zur zeitaufgelösten Anzeige von stereoskopischen Bilddaten ausgebildetes Anzeigemittel auf. Bei dem Anzeigemittel handelt es sich bevorzugt um ein Anzeigemittel, das zur Anzeige von Bilddaten mit verschiedenen (beispielsweise orthogonalen) Polarisationen ausgebildet ist, in Kombination mit einer Polarisationsbrille. Ebenfalls bevorzugt ist eine Kodierung der stereoskopischen Bilddaten mittels Farbfilterung und eine Kombination mit einer 3D-Brille mit Farbfiltern. Bei dem Anzeigemittel kann es sich jedoch auch um einen 3D Bildschirm, beispielsweise einen Lichtfeldmonitor oder dergleichen, handeln. Bei dem Anzeigemittel handelt es sich bevorzugt um einen oder mehrere Bildschirme, beispielsweise um zumindest einen Bildschirm eines Operationsmikroskops, um einen in einem Operationsraum fest installierten Bildschirm oder um ein Head-Mounted-Display (HMD), beispielsweise eine Videobrille. Bei dem Bildschirm handelt es sich vorzugsweise um einen 4K und/oder 8K fähigen Bildschirm.

Das System gemäß der vorliegenden Offenbarung weist ferner eine Steuereinheit auf, die mit dem OCT-System und dem Anzeigemittel verbunden ist, insbesondere zur ein- oder wechselseitigen Datenübertragung. Die Steuereinheit ist dazu eingerichtet, das OCT-System zum Erfassen eines zeitaufgelösten OCT-Signals eines ausgewählten Sichtfelds (*region of interest - ROI*) einer Probe anzusteuern. Bei der Probe handelt es sich beispielsweise um ein Operationsgebiet eines Patienten, bei ophthalmologischen Operationen insbesondere um ein Auge. Bei der Probe kann es sich jedoch auch um beliebige andere Operationsgebiete handeln, wie beispielsweise um Hirngewebe in der Neurochirurgie, um im HNO-Bereich befindliches Gewebe bei der HNO-Chirurgie oder um Zahnfleisch, Zahnstein oder Zahnnerven bei der Dentalchirurgie. Ebenso kann es sich um beliebige andere Gewebe oder Präparate (in vivo, in vitro oder in situ) handeln. Das Sichtfeld wird bevorzugt durch einen Nutzer ausgewählt. Das zeitaufgelöste OCT-Signal wird bevorzugt erfasst, indem ein Lichtsignal erzeugt und mittels des Interferometers teilweise als Probenstrahl auf die Probe gelenkt und in dem Interferometer mit einem ebenfalls aus dem Lichtsignal erzeugten Referenzstrahl zur Überlagerung gebracht wird, um ein Interferenzmuster zu erzeugen.

Die Steuereinheit des Systems gemäß der vorliegenden Offenbarung ist ferner dazu ausgebildet, anhand des erfassten zeitaufgelösten OCT-Signals, einer vorgebbaren virtuellen Blickrichtung und eines Stereowinkels α ein zeitaufgelöstes erstes OCT-Bild und ein zeitaufgelöstes zweites OCT-Bild zu ermitteln. Bei dem OCT-Signal handelt es sich um ein Interferenzsignal, wobei die Modulation der Einhüllenden des Interferenzsignals Reflektionseigenschaften der Probe kodiert. Mittels des Scanmechanismus kann die Probe zweidimensional in einer über den Gangunterschied eingestellten Probentiefe abgerastert werden. Anhand des eingesetzten Scanmechanismus, dem eingesetzten Mittel zum Auswählen beziehungsweise Erzeugen des Gangunterschieds, beispielsweise eines verstellbaren Spiegels im Referenzstrahl, einem optischen Gitter vor dem Detektor oder einer durchstimmbaren breitbandigen Lichtquelle, und der Wiederholrate des Detektors ergibt sich für das zeitaufgelöste OCT-Signal eine Taktfrequenz (Bildwiederholrate). Die Steuereinheit ermittelt anhand des OCT-Signals rechnerisch zeitaufgelöste OCT-Bilder, beispielsweise mittels Volumen-Rendering, Ray-Tracing und/oder Ray-Marching. Dazu ermittelt die Steuereinheit zunächst eine virtuelle Blickrichtung und berücksichtigt diese virtuelle Blickrichtung bei dem Ermitteln des OCT-Bilds mittels Volumen-Rendering, Ray-Tracing und/oder Ray-Marching. Somit wird durch das System gemäß der vorliegenden Offenbarung ein OCT-Bild anhand des OCT-Signals und einer vorgebbaren virtuellen Blickrichtung ermittelt, wodurch ein durch das OCT-System bereitgestellter Freiheitsgrad zur perspektivischen Ermittlung des OCT-Bildes vorteilhaft nutzbar ist. Die virtuelle Blickrichtung ist dabei prinzipiell von einer tatsächlichen Blickrichtung eines Nutzers verschieden. Die virtuelle Blickrichtung stellt vielmehr eine durch den Nutzer wie unten beschrieben vorgebbare und/oder anhand von Geräteparametern wie untenstehend ermittelbare Raumrichtung dar, die als Berechnungsgrundlage des OCT-Bilds dient.

Die Steuereinheit des Systems gemäß der vorliegenden Offenbarung ist ferner dazu eingerichtet, die ermittelten zeitaufgelöste OCT-Bilder auf dem Anzeigemittel stereoskopisch darzustellen. Die Anzeige erfolgt dabei vorteilhaft in der virtuellen Blickrichtung. Die virtuelle Blickrichtung ist dabei bevorzugt für einen definierten Zeitraum eingestellt. Ebenfalls bevorzugt ist die virtuelle Blickrichtung in Form einer Sequenz eingestellt und wechselt beispielsweise automatisch nach Ablauf einer vordefinierten Zeit, beim Erfassen einer vordefinierten Nutzereingabe (beispielsweise ein Betätigen eines Fußpedals), beim Ermitteln einer Zustands- oder Positionsänderung eines medizintechnischen Instruments, beim Ermitteln einer Änderung eines Geräteparameters und/oder beim Erkennen eines Wechsels einer Phase einer durchgeführten Operation. Ebenfalls bevorzugt folgt die virtuelle Blickrichtung kontinuierlich oder diskontinuierlich einer Nutzereingabe, beispielsweise anhand einer erfassten tatsächlichen Blickrichtung des Nutzers. So kann beispielsweise eine Neigung des Kopfes des Nutzers erfasst werden und das OCT-Bild mit einer entsprechenden Neigung erstellt und auf dem Anzeigemittel dargestellt werden. Das System gemäß der vorliegenden Offenbarung ermöglicht somit vorteilhaft eine optimale Visualisierung des OCT-Signals entsprechend der Bedürfnisse des Nutzers, wodurch die Potentiale des OCT-Signals optimal ausgenutzt werden und dem Nutzer ein maximaler Informationsgehalt zur Verfügung gestellt wird.

Die erfindungsgemäße Steuereinheit ist mithin dazu eingerichtet, anhand des zeitaufgelösten OCT-Signals, der virtuellen Blickrichtung und des Stereowinkels ein zeitaufgelöstes erstes OCT-Bild und ein zeitaufgelöstes zweites OCT-Bild zu ermitteln und das erste OCT-Bild und das zweite OCT-Bild auf dem Anzeigemittel stereoskopisch darzustellen. Der Stereowinkel ist dabei maßgeblich für den Tiefeneindruck bei der Bildvisualisierung auf dem Anzeigemittel. Bei einer Stereokamera entspricht der Stereowinkel dem Winkel zwischen den optischen Achsen der einzelnen Kameras der Stereokamera und hängt somit vom Abstand der Kameras zueinander als auch vom Arbeitsabstand der Kameras zum Objekt (Probe) ab. Bei der Erzeugung der stereoskopischen OCT-Bilder wird ein solcher Stereowinkel rechnerisch zugrunde gelegt, was eine variable Einstellung des Stereowinkels ermöglicht. Ein großer Stereowinkel entspricht in der Regel einer starken Tiefenwahrnehmung (wie bei der Wahrnehmung naher Objekte durch das menschliche Auge) und ein kleiner Stereowinkel in der Regel einer geringen Tiefenwahrnehmung (wie bei der Wahrnehmung ferner Objekte durch das menschliche Auge). Somit ermöglicht das System gemäß der vorliegenden Offenbarung vorteilhaft eine Darstellung der OCT-Bilder mit variablem Tiefeneindruck.

Die Steuereinheit ist ferner dazu eingerichtet, eine Phase einer durchgeführten Operation zu ermitteln und den Stereowinkel anhand der Phase der durchgeführten Operation zu ermitteln. Hierzu ist die Steuereinheit bevorzugt mit einem Speicher verbunden, in dem ein trainierter Algorithmus des maschinellen Lernens, beispielsweise ein neurales Netzwerk (CNN) oder dergleichen abgelegt ist. Dieser Algorithmus wurde bevorzugt mittels einer Vielzahl von Videobilddaten und/oder OCT-Bildern beziehungsweise Bildsignalen und/oder OCT-Signalen trainiert, denen im Training eine korrespondierende Phase einer OP als Klassifikation zugeordnet worden ist. Demzufolge ist der trainierte Algorithmus in der Lage anhand von Videobilddaten und/oder OCT-Bildern beziehungsweise Bildsignalen und/oder OCT-Signalen selbständig eine Phase einer Operation als Klassifikation zu erkennen. Bevorzugt ist die Steuereinheit gemäß dieser Ausführungsform dazu ausgebildet, einen zu der ermittelten Phase der Operation korrespondierenden Stereowinkel auszuwählen. Die für verschiedene Phasen der Operation geeignet Stereodaten sind bevorzugt in einem Look-up-Table (LUT) in einem Speicher hinterlegt oder werden ebenfalls mit einem Algorithmus des maschinellen Lernens ermittelt. Dieser kann beispielsweise trainiert werden, indem einer Vielzahl von Videobilddaten und/oder OCT-Bildern beziehungsweise Bildsignalen und/oder OCT-Signalen während des Trainings eine korrespondierende Phase einer OP und/oder ein korrespondierender Stereowinkel als eine oder mehrere Klassifikation zugeordnet worden ist.

Bei einer Kataraktoperation können die Operationsphasen beispielsweise umfassen: Ruhezustand, Inzision, Injektion des ophthalmischen viskochirurgischen Geräts (OVD), Kapsulorhexis, Hydrodissektion, Phakoemulsifikation, Spülung/Aspiration, Implantation der Intraokularlinse, Schließen/Befeuchten der Wunde, Nichtoperation. Bei einer refraktiven Operation können die Operationsphasen beispielsweise umfassen: Leerlauf, Andocken, Applanation, Anlegen des Auges/CG-Drehung, Linsenschnitt, Linsenseitenschnitt, Kappenschnitt, Kappenseitenschnitt, Freigeben des Auges, Übergang zum OPMI, Positionierung des OPMI, Eröffnung der Inzision, Festlegung der Ebenen, Abtrennung des Kappenbetts, Abtrennung des Linsenbetts, Entfernung und/oder Inspektion der Linsen, Abwischen, Spülen, Spaltlampe, Entfernung des Spekulums. Bei einem zahnärztlichen Eingriff können die chirurgischen Phasen beispielsweise umfassen: Zugang, Exstirpation, Debridement, Trocknung, Obturation, Restauration. Es ist zu beachten, dass alle oder nur einige dieser Phasen Teil der entsprechenden Operation sein können und dass auch weitere Operationsphasen vorhanden sein und/oder einige Phasen ausgelassen werden können.

In einer bevorzugten Ausführungsform des Systems gemäß der vorliegenden Offenbarung ist die virtuelle Blickrichtung durch einen Azimuthwinkel und einen Höhenwinkel relativ zur optischen Achse des OCT-Systems definiert. Die optische Achse des OCT-Systems entspricht dabei bevorzugt einer optischen Achse eines (nachfolgend erläuterten) operationsmikroskopischen Systems des Systems gemäß der vorliegenden Offenbarung und/oder steht bevorzugt senkrecht auf einer Oberfläche der Probe. Der Azimuthwinkel bezeichnet bevorzugt einen Winkel zwischen 0° und 360° in einer zu der optischen Achse senkrechten Ebene, bevorzugt in der Ebene der Probenoberfläche. Der Azimuthwinkel ist ein ebener Winkel und wird zwischen einem Nullpunkt und einer Projektion der virtuellen Blickrichtung in diese Ebene gemessen. Der Azimuthwinkel definiert somit eine virtuelle Blickrichtung im engeren Sinn. Der Höhenwinkel bezeichnet bevorzugt einen Winkel zwischen 0° und 90° in einer die optische Achse enthaltenden Ebene, bevorzugt in einer zu der Probenoberfläche orthogonalen Ebene. Der Höhenwinkel ist ein ebener Winkel und wird beispielsweise zwischen der virtuellen Blickrichtung und der Probenoberfläche gemessen. Der Höhenwinkel definiert somit einen vertikalen Blickwinkel.

In einer ferner bevorzugten Ausführungsform des Systems gemäß der vorliegenden Offenbarung ist die Steuereinheit dazu eingerichtet, das zeitaufgelöste OCT-Bild als ein dreidimensionales und aus der virtuellen Blickrichtung perspektivisches Volumenbild zu ermitteln. Neben der Darstellung von OCT-Bildern in *"en-face"* Darstellung, also in einer Draufsicht, ermöglicht die Steuereinheit somit auch die perspektivische Darstellung der OCT-Signale. Ebenfalls bevorzugt ist die Steuereinheit ferner dazu ausgebildet, die OCT-Signale in Form von Schnittbildern in einer Höhenrichtung der Probe (sogenannte B-Scans) darzustellen. Die Darstellung der zeitaufgelösten OCT-Bilder als dreidimensionales und perspektivisches Volumenbild aus verschiedenen Perspektiven ermöglicht es vorteilhaft, maximale Tiefeninformation über das Objekt zu erhalten. Insbesondere können sowohl die gerade Darstellung aus der Vogelperspektive (Draufsicht, "en face"), diese aus verschiedenen virtuellen Blickrichtungen (Azimuth- und Höhenwinkel), als auch Schnittbilder (B-Scans) entlang verschiedener Schnittlinien sinnvoll sein, beispielsweise um während verschiedener Phasen eines Eingriffs die jeweils relevanten Informationen über die Probe zu erhalten.

Mit anderen Worten kann, insbesondere bei medizinischen (beispielsweise ophthalmologischen) Anwendungen, die sinnvollste virtuellen Blickrichtung je nach Applikation und OP-Situation variieren. So kann es sinnvoll sein, die zeitaufgelösten OCT-Bilder (4D-iOCT) zunächst aus der technischen Blickrichtung eines Videos eines Operationsmikroskops darzustellen (wobei die technische Blickrichtung beispielsweise anhand eines Geräteparameters ermittelt ist), um dem Nutzer den besten Überblick zu bieten. Im Laufe der weiteren Anwendung beziehungsweise Operation können jedoch andere, beispielsweise seitliche, virtuelle Blickrichtungen eine weitaus bessere Tiefeninformation gewährleisten. Die sich dadurch ergebende Perspektive entspricht zudem eher der natürlichen Perspektive des menschlichen Blickes mit unbewaffnetem Auge und bietet auch daher einen Vorteil für die mikrochirurgische Arbeit.

Das System gemäß der vorliegenden Offenbarung weist bevorzugt eine Schnittstelle zum Erfassen einer Nutzereingabe auf. Bei der Schnittstelle handelt es sich bevorzugt um einen Handschalter oder um einen Fußschalter. Ebenso bevorzugt handelt es sich bei der Schnittstelle um ein Mittel zum Erkennen einer Kopf- und/oder Augenbewegung beziehungsweise einer tatsächlichen Blickrichtung, beispielsweise integriert in eine Videobrille oder in ein *Head-Mounted-Display,* HMD. Die Schnittstelle kann ferner zum Erfassen von Sprachbefehlen ausgebildet sein und hierfür zumindest ein Mikrofon umfassen. Ebenso bevorzugt handelt es sich bei der Schnittstelle um eine Tastatur, einen Joystick, eine Maus, einen Touchscreen oder eine Kombination davon.

Gemäß dieser bevorzugten Ausführungsform ist die Steuereinheit ferner dazu ausgebildet, die Schnittstelle zum Erfassen einer Nutzereingabe anzusteuern. Ferner bevorzugt geht der Nutzereingabe eine Eingabeaufforderung voraus, die beispielsweise mittels des Ausgabemittels an den Nutzer ausgegeben wird. Ebenfalls bevorzugt ist die Nutzereingabe eine Auswahl aus einer Mehrzahl vordefinierter Eingabemöglichkeiten, welche beispielsweise eine virtuelle Blickrichtung, eine durchzuführende Operation und/oder einen durchzuführenden Eingriff spezifizieren. Ebenfalls bevorzugt handelt es sich bei der Nutzereingabe um eine vom Nutzer innerhalb eines vorgegebenen Rahmens frei definierbare Nutzereingabe. Ebenfalls bevorzugt ist die Steuereinheit dazu ausgebildet, den Nutzer bei der Nutzereingabe zu unterstützen, beispielsweise durch Anzeige einer Mehrzahl von Dialogen zur gezielten Abfrage von bestimmten Nutzereingaben.

Gemäß dieser Ausführungsform ist die Steuereinheit ferner dazu ausgebildet, die virtuelle Blickrichtung anhand der Nutzereingabe zu ermitteln. Der Nutzer kann somit vorteilhaft die als optimal erachtete virtuelle Blickrichtung gezielt auswählen. Besonders bevorzugt wird mittels der Schnittstelle eine Nutzereingabe erfasst, welche den Azimuthwinkel und den Höhenwinkel spezifiziert, beispielsweise nach einer entsprechenden Eingabeaufforderung an den Nutzer. Ebenfalls bevorzugt werden Azimuthund Höhenwinkel aus der Nutzereingabe abgeleitet, beispielsweise aus einer mittels Kopf- und/oder Augensteuerung durch den Nutzer unmittelbar spezifizierten Blickrichtung.

Alternativ oder zusätzlich weist das System ferner eine zum Erfassen eines Geräteparameters ausgebildete Geräteschnittstelle auf. Bei der Geräteschnittstelle handelt es sich vorzugsweise um eine Schnittstelle zum Anschluss eines operationsmikroskopischen Systems wie untenstehend noch im Detail beschrieben. Ebenfalls bevorzugt handelt es sich bei der Geräteschnittstelle aber auch um eine Schnittstelle zum Anschluss eines beliebigen anderen bildgebenden Systems. Bei der Geräteschnittstelle kann es sich ferner um eine Schnittstelle zum Anschließen eines medizintechnischen Instruments handeln. Ebenso bevorzugt ist über die Geräteschnittstelle ein System zum Tracking eines medizinischen oder medizintechnischen Instruments anschließbar, wobei es sich bei dem Trackingsystem auch um das operationsmikroskopische System oder ein anderes bildgebendes System handeln kann. Bei dem medizintechnischen Instrument handelt es sich beispielsweise um einen Pointer, eine Sonde, eine Pinzette, eine Ahle, einen Phako-Tip, ein Endoskop, eine Endo-LED eines Greifers oder dergleichen. Gemäß dieser Ausführungsform ist die Steuereinrichtung bevorzugt dazu eingerichtet mit der Geräteschnittstelle zu kommunizieren, insbesondere bidirektional zu kommunizieren und ferner dazu, die Geräteschnittstelle zum Erfassen eines Geräteparameters eines mittels der Schnittstelle angeschlossenen Geräts anzusteuern.

Gemäß dieser Ausführungsform ist die Steuereinheit ferner dazu ausgebildet, die virtuelle Blickrichtung anhand eines mittels der Geräteschnittstelle erfassten Geräteparameters zu ermitteln. Bevorzugt können anhand der Geräteparameter eines angeschlossenen operationsmikroskopischen Systems oder anderen bildgebenden Systems eine technische Blickrichtung des operationsmikroskopischen Systems oder anderen bildgebenden Systems ermittelt werden und kann die virtuelle Blickrichtung gleich der technischen Blickrichtung gesetzt werden. Dies ermöglicht eine multimodale Bilderfassung und -darstellung, wobei eine virtuelle Blickrichtung des OCT-Bilds vorteilhaft mit einer (technischen) Blickrichtung der anderen Bilddaten übereinstimmt.

Ebenfalls bevorzugt beschreibt der Geräteparameter eine Lage, eine Position und/oder einen Zustand eines angeschlossenen oder eines mittels des Trackingsystems getrackten medizintechnischen (oder eines getrackten medizinischen) Instruments und wird die virtuelle Blickrichtung anhand der Lage, der Position und/oder des Zustand des medizintechnischen (medizinischen) Instruments ermittelt. So kann die virtuelle Blickrichtung beispielsweise entsprechend der Lage eines Pointers (gegebenenfalls entlang der Längsachse des Pointers) oder entsprechend des Zustands eines Greifers ermittelt werden. Dabei kann die Raumlage des medizintechnischen Instruments durch das Instrument selbst ermittelt werden und/oder kann die Raumlage des medizinischen oder medizintechnischen Instruments über ein Trackingsystem ermittelt werden. Ein medizinisches Instrument muss dabei über keinerlei eigene Elektronik verfügen, sondern können dessen Eigenschaften beispielsweise allein durch das Tracking System, gegebenenfalls unterstützt durch auf dem medizinischen Instrument angeordnete Marker, ermittelt werden. Ebenso bevorzugt wird die (Bild-)Erkennung des medizintechnischen Instruments durch eine Nutzereingabe initiiert, wobei die Nutzereingabe auf dem Instrument selbst oder anderweitig erfolgen kann.

Ein über die Geräteschnittstelle empfangene Geräteparameter zum Zustand eines angeschlossenen medizintechnischen Instruments umfasst bevorzugt auch die Betätigung eines Eingabemittels des medizintechnischen Geräts, so dass in diesem Fall der Geräteparameter gleichzeitig auch eine Nutzereingabe darstellt. Ebenfalls bevorzugt werden verschiedene Geräteparameter gemeinsam berücksichtigt, wie eine Lage des medizin-technischen Instruments (beispielsweise ermittelt durch das Instrument selbst und/oder durch ein Trackingsystem) und die Betätigung eines Eingabemittels des medizintechnischen Instruments. Somit ist beispielsweise eine Sequenz virtueller Blickrichtungen entsprechend einer Sequenz von Lagen des Instruments vorteilhaft erfassbar. Ein das medizinische oder medizintechnische Instrument beschreibender Geräteparameter kann über die Geräteschnittstelle vom Instrument selbst oder von einem Trackingsystem empfangen werden. Ferner bevorzugt werden Azimuth- und Höhenwinkel der virtuellen Blickrichtung, wie obenstehend beschrieben, aus einem empfangenen Geräteparameter abgeleitet.

In einer bevorzugten Ausführungsform des Systems gemäß der vorliegenden Offenbarung weist ein OCT-Signal eine Vielzahl von (ersten) Tupeln auf, die jeweils ein Volumenelement der Probe und eine Streuintensität umfassen (beziehungsweise repräsentieren). Das Volumenelement der Probe ist dabei bevorzugt durch drei Raumkoordinaten (beispielsweise *x*, *y* und *z*) repräsentiert und kann beispielsweise als ein Probenvoxel interpretiert werden. Neben der Streuintensität kann das Tupel weitere Werte aufweisen. Gemäß dieser Ausführungsform weist das Anzeigemittel eine Vielzahl von Pixeln auf und ist die Steuereinheit dazu eingerichtet, die zeitaufgelösten OCT-Bilder anhand der (ersten) Tupel, anhand der Auflösung des Anzeigemittels und anhand der virtuellen Blickrichtung so zu ermitteln, dass bestimmte Pixel zu bestimmten Volumenelementen korrespondieren, sprich dass bestimmte Pixel bestimmte Volumenelemente der Probe anzeigen. Mit anderen Worten ermittelt die Steuereinheit eine Zuordnung von Pixeln des Anzeigemittels und Volumenelementen der Probe. Diese Zuordnung kann dabei von weiteren Einstellungen abhängen, wie beispielsweise einer Rasterauflösung des eingesetzten Scanmechanismus, ist aber für eine gegebene Auswahl von Einstellungen, wie virtuelle Blickrichtung, Zoomstufe und Stereowinkel, bevorzugt zeitlich konstant. Die Steuereinheit realisiert somit eine örtliche Registrierung zwischen Pixeln des Anzeigemittels und den OCT-Signalen beziehungsweise den ermittelten OCT-Bildern.

Ferner bevorzugt ist die Steuereinheit dazu eingerichtet, die durch das OCT-System erfassten OCT-Signale anhand von Erfassungsparametern des OCT-Systems örtlich zu registrieren. Eine örtliche Registrierung dieser Signale bezeichnet dabei eine korrekte Verknüpfung dieser Signale mit einem Referenzkoordinatensystem, beispielsweise dem Koordinatensystem des Patienten während eines Eingriffs, und ermöglicht eine eindeutige Abbildung (Mapping) von Koordinaten des Patientenraumes auf entsprechende Koordinaten des Signalraumes. Eine Registrierung der Signale erfordert bevorzugt eine Kalibrierung des OCT Systems. Die Erfassungsparameter des OCT-Systems weisen bevorzugt Kalibrierungsparameter auf. Die Erfassungsparameter des OCT-Systems berücksichtigen bevorzugt den Scanmechanismus und/oder den Detektor des OCT-Systems. Anhand der örtlichen Registrierung anhand der Erfassungsparameter können vorteilhaft an definierten Koordinaten des Patientenraums befindliche Strukturen des Patienten an den entsprechenden Koordinaten im Bildraum der OCT-Bilder korrekt dargestellt werden, insbesondere mit korrekten Relativlagen zueinander.

In einer besonders bevorzugten Ausführungsform des Systems gemäß der vorliegenden Offenbarung weist dieses ferner ein zum Erfassen eines zeitaufgelösten Bildsignals des ausgewählten Sichtfelds der Probe ausgebildetes operationsmikroskopisches System auf. Das operationsmikroskopische System weist bevorzugt eine Optik, insbesondere zum Erfassen von der Probe reflektierten oder rückgestreuten Lichts, beispielsweise sichtbaren Lichts, auf. Die Optik umfasst beispielsweise eine Objektivlinse und ein Okular, kann darüber hinaus jedoch weitere Komponenten umfassen, insbesondere weitere Linsen, Spiegel, Strahlteiler und/oder dergleichen. Das operationsmikroskopische System weist ferner einen Bildsensor auf, der zum Erfassen eines zeitaufgelösten Bildsignals des ausgewählten Sichtfelds (*region of interest - ROI*) einer Probe ausgebildet ist. Die Optik und der Bildsensor sind bevorzugt integriert ausgebildet, beispielsweise als Teile einer Kamera eines Operationsmikroskops. Gemäß dieser Ausführungsform ist die Steuereinheit ferner dazu ausgebildet und eingerichtet zu dem erfassten zeitaufgelösten Bildsignal korrespondierende Videobilddaten zu ermitteln. Bei dem zeitaufgelösten Bildsignal handelt es sich insbesondere um eine Vielzahl von Oberflächenelementen der Probe zugeordneten Signalen, die sequentiell oder simultan für ein bestimmtes Raster der Probenoberfläche erfasst sind, wobei das Raster durch einen Scanmechanismus und/oder den Bildsensor bestimmt ist. Das zeitaufgelöste Bildsignal weist ferner eine Taktfrequenz (Bildwiederholrate) auf, die durch einen Scanmechanismus und/oder den Bildsensor bestimmt ist. Die Steuereinheit erzeugt aus diesem Bildsignal Videobilddaten mit einem Raster (Auflösung) und einer Bildwiederholrate die zur Darstellung auf dem Anzeigemittel geeignet sind. Die Steuereinheit ist ferner dazu eingerichtet die Videobilddaten mittels des Anzeigemittels darzustellen.

In dem System gemäß der vorliegenden Offenbarung weist ein Bildsignal bevorzugt eine Vielzahl von (zweiten) Tupeln auf. Dabei umfasst (beziehungsweise repräsentiert) jedes (zweite) Tupel ein Oberflächenelement der Probe und zumindest einen Graustufenwert. Das Oberflächenelement der Probe ist dabei bevorzugt durch zwei laterale Raumkoordinaten (beispielsweise x und y) repräsentiert und kann beispielsweise als ein Probenpixel interpretiert werden. Neben dem Graustufenwert, der sich letztlich aus einer erfassten Intensität ergibt, kann jedes (zweite) Tupel darüber hinaus auch Farbwerte auffassen, beispielsweise bei der Erfassung von Intensitäten für verschiedene Farben mittels dem Bildsensor vorgeschalteten Farbfiltern. Gemäß dieser Ausführungsform ist die Steuereinheit zudem dazu eingerichtet, die Videobilddaten anhand der (zweiten) Tupel und anhand einer Auflösung des Anzeigemittels so zu ermitteln, dass bestimmte Pixel bestimmte Oberflächenelemente der Probe anzeigen. Mit anderen Worten ermittelt die Steuereinheit eine (zweite) Zuordnung von Pixeln des Anzeigemittels und Oberflächenelementen der Probe zusätzlich zu der (ersten) Zuordnung von Pixeln des Anzeigemittels und Volumenelementen der Probe. Diese (zweite) Zuordnung kann dabei von weiteren Einstellungen abhängen, wie beispielsweise einer Zoomstufe des operationsmikroskopischen Systems, ist aber für gegebene Einstellungen bevorzugt zeitlich konstant. Die (zweite) Zuordnung korrespondiert bevorzugt zu der (ersten) Zuordnung. Die Steuereinheit realisiert somit auch eine örtliche Registrierung zwischen Pixeln des Anzeigemittels und Bildsignalen (Videobilddaten) des operationsmikroskopischen Systems.

Ferner bevorzugt ist die Steuereinheit ferner dazu eingerichtet, die durch das operationsmikroskopische System erfassten Bildsignale anhand von Erfassungsparametern des operationsmikroskopischen Systems örtlich zu registrieren. Eine Registrierung der Signale erfordert bevorzugt eine Kalibrierung des operationsmikroskopischen Systems. Die Erfassungsparameter des operationsmikroskopischen Systems umfassen somit bevorzugt Kalibrierungsparameter und/oder optische Einstellungen des operationsmikroskopischen Systems, wie beispielsweise eine Fokuslänge und/oder eine Zoomstufe einer eingesetzten Optik (Kamera). Darüber hinaus umfassen die Erfassungsparameter bevorzugt auch einen Satz intrinsischer Parameter des operationsmikroskopischen Systems. Die intrinsischen Parameter bestimmen dabei einen Zusammenhang zwischen dem Koordinatensystem eines Bildsignals und dem Koordinatensystem des zugehörigen bildgebenden Sensors. Die Art der intrinsischen Parameter hängt dabei insbesondere von der Art des eingesetzten bildgebenden Sensors ab, wobei mit bildgebendem Sensor hier sowohl der eigentliche Sensor als auch die verwendete Optik bezeichnet ist. Die intrinsischen Parameter umfassen bei einer einer Kamerakalibrierung nach Tsai beispielsweise eine effektive Brennweite, die Koordinaten eines Bildhauptpunktes (Zentrum der Verzerrung) eines Bildsignals, einen ersten Skalierungsfaktor und/oder einen ersten radialen Linsenfehlerkoeffizienten (Verzerrungskoeffizient). Alternativ zu den vorgenannten intrinsischen Parametern einer Kamerakalibrierung nach Tsai können auch andere intrinsische Parameter, beispielsweise für eine Kamerakalibrierung nach Zhang verwendet werden (vgl. beispielsweise *"*A practical comparison between Zhang's and Tsai's calibration approaches", Li et al., Proceedings of the 29th International Conference on Image and Vision Computing New Zealand, November 2014 Pages 166-171, DOI:10.1145/2683405.2683443).

Die örtliche Registrierung der OCT-Signale gemeinsam mit der örtlichen Registrierung der Bildsignale ermöglicht vorteilhaft das Erzeugen und Darstellen der OCT-Bilder und der Videobilddaten, so dass das erzeugte zeitaufgelöste OCT-Bild zumindest zu einem Abschnitt der dargestellten Videobilddaten korrespondiert. Bevorzugt wird ein OCT-Signal von dem gesamten Sichtfeld erfasst und wird ein OCT-Bild von zumindest einem Teil des Sichtfelds erstellt. Ebenso bevorzugt wird ein OCT-Signal von einem Abschnitt des Sichtfelds erfasst und wird ein OCT-Bild von zumindest einem Teil des Abschnitts des Sichtfelds erstellt. Die Steuereinheit ist ferner dazu ausgebildet, das zeitaufgelöste OCT-Bild an der Position des Abschnitts der Videobilddaten auf dem Anzeigemittel darzustellen. Bevorzugt werden Videobilddaten und ein OCT-Bild von dem gesamten Sichtfeld erstellt und jeweils auf dem gesamten Anzeigemittel dargestellt. Ebenso bevorzugt wird ein OCT-Bild von einem Abschnitt des Sichtfelds erzeugt und an der Position der zu diesem Abschnitt des Sichtfelds korrespondierenden Videodaten auf dem Anzeigemittel dargestellt. Mit anderen Worten werden Videobilddaten und OCT-Bilder, die zu demselben Abschnitt der Probe korrespondieren an derselben Stelle des Anzeigemittels dargestellt. Das System gemäß der vorliegenden Offenbarung ermöglicht somit eine nahtlose Integration von Videobilddaten und OCT-Bildern auf dem Anzeigemittel, wodurch einem Nutzer eine einfachere Betrachtung der multimodalen Bilddaten ermöglicht wird. So können die multimodalen Bilddaten betrachtet werden, ohne den Kopf oder die Augen zu bewegen, was sich insbesondere bei Bildgebung während operativer Eingriffe vorteilhaft auf die Aufmerksamkeit des Operateurs auswirkt.

Die Steuereinheit des Systems ist bevorzugt dazu eingerichtet ist, die Videobilddaten und das zeitaufgelöste OCT-Bild gleichzeitig auf dem Anzeigemittel darzustellen. Dies ermöglicht besonders vorteilhaft die gleichzeitige Berücksichtigung beider Bildmodalitäten durch einen Anwender. Um dennoch eine Unterscheidung der verschiedenen Bilddaten zu ermöglichen, ist die Steuereinheit ferner bevorzugt dazu eingerichtet, die Videobilddaten mit einer ersten Transparenzstufe und das zeitaufgelöste OCT-Bild mit einer zweiten Transparenzstufe darzustellen. Dabei unterscheiden sich die erste und die zweite Transparenzstufe bevorzugt. Ebenfalls bevorzugt variieren die erste und zweite Transparenzstufe zeitlich. Beispielsweise wird zunächst das Bildsignal mit einer Transparenz von 0% dargestellt, während das OCT-Bild mit einer Transparenz von 100% dargestellt wird. Im zeitlichen Verlauf wird die Transparenz des Bildsignals dann kontinuierlich von 0% auf 100% eingestellt, während zeitgleich die Transparenz des OCT-Bildes von 100% auf 0% eingestellt wird. Somit wird ein kontinuierlicher Übergang zwischen der Darstellung des Videobildes und der des OCT-Bildes gewährleistet.

In einer ebenfalls bevorzugten Ausführungsform des Systems gemäß der vorliegenden Offenbarung ist die Steuereinheit ferner dazu ausgebildet, die Videobilddaten und OCT-Bilder mittels Bildanalyse örtlich zu registrieren. Dabei bezeichnet eine örtliche Registrierung dieser Bilddaten eine korrekte Verknüpfung dieser Bilder in einem gemeinsamen Bildkoordinatensystem. Die Registrierung anhand der Bilddaten ermöglicht somit eine relative Verknüpfung der Bilddaten für eine möglichst deckungsgleiche Abbildung gleicher Strukturen. Beispielsweise können Struktur- oder Gewebegrenzen in den Videobilddaten und den OCT-Bildern mittels Bildanalyse, beispielsweise Kantenerkennung oder dergleichen, erkannt und miteinander verglichen werden. Durch translatorische Verschiebung, Rotation und/oder Skalierung können diese Strukturen dann auf dem Anzeigemittel miteinander in Überlagerung gebracht werden. Bevorzugt erfolgt die örtliche Registrierung der Bilddaten zusätzlich zu einer örtlichen Registrierung der erfassten Signale, wie obenstehend beschrieben.

In einer ebenfalls bevorzugten Ausführungsform des Systems gemäß der vorliegenden Offenbarung ist die Steuereinheit dazu eingerichtet, die Videobilddaten und das zeitaufgelöste OCT-Bild sequentiell auf dem Anzeigemittel darzustellen. Mit anderen Worten wird, zumindest an einem bestimmten Ort des Anzeigemittels, zu jeder Zeit nur eines von Videobilddaten und OCT-Bildern dargestellt. Dies ermöglicht vorteilhaft eine deutliche Unterscheidung zwischen Videobilddaten und OCT-Daten und ebenso vorteilhaft eine gleichzeitige Darstellung der verschiedenen Bilddaten an verschiedenen Orten des Anzeigemittels.

Besonders bevorzugt ist die Steuereinheit dazu eingerichtet, die Videobilddaten und das zeitaufgelöste OCT-Bild mit gleicher Vergrößerung, gleicher Perspektive und/oder gleichem Stereowinkel darzustellen. Dies ermöglicht bei einer gleichzeitigen Darstellung beider Videobilddaten an einem selben Ort des Anzeigemittels bevorzugt eine perfekte Überlagerung beider Bilddaten und kann vorteilhaft eine Darstellung mit optimiertem Kontrast ermöglichen. Bei einer sequentiellen Darstellung erfolgt bevorzugt am Übergang zwischen den örtlich registrierten Bilddaten eine Darstellung mit gleicher Vergrößerung, gleicher Perspektive und/oder gleichem Stereowinkel. Somit erfolgt ein flüssiger Übergang zwischen der Darstellung der Bilddaten. Bei den Videobilddaten ist lediglich eine Darstellung der Oberfläche (Draufsicht) möglich. Dies entspricht beispielsweise einem *"en-face"* OCT-Bild. Sobald ein Übergang von Videobilddaten zu OCT-Bild erfolgt ist, ist ferner bevorzugt die Vergrößerung, Perspektive und/oder der Stereowinkel anpassbar, um vorteilhaft eine verbesserte Ansicht mit optimaler Tiefenwahrnehmung zu ermöglichen. Insbesondere kann von der initialen *"en-face"* Ansicht auf eine perspektivische Darstellung, beispielsweise durch einen kontinuierlichen Übergang der virtuellen Blickrichtung, oder auf eine Schnittansicht (OCT-B-Scan) gewechselt werden. Somit wird in verschiedenen Phasen vorteilhaft die perfekte Ansicht gewährleistet.

In einer bevorzugten Ausführungsform des Systems gemäß der vorliegenden Offenbarung ist die Steuereinheit dazu eingerichtet, den Stereowinkel basierend auf einer mittels der Schnittstelle erfassten Nutzereingabe zu ermitteln. Somit kann ein Nutzer, beispielsweise ein Operateur einen Stereowinkel vorteilhaft individuell anpassen, so dass durch die Berücksichtigung der Nutzereingabe beim Ermitteln des Stereowinkels vorteilhaft auch subjektiven Präferenzen Rechnung getragen werden kann.

In einer ferner bevorzugten Ausführungsform des Systems gemäß der vorliegenden Offenbarung ist die Steuereinheit ferner dazu eingerichtet, den Stereowinkel anhand optischer Parameter des operationsmikroskopischen Systems zu ermitteln. Die optischen Parameter des operationsmikroskopischen Systems werden dabei in Form von Metadaten durch die Steuereinheit des Systems gemäß der vorliegenden Offenbarung erfasst beziehungsweise eingelesen. Somit ist die Steuereinheit vorteilhaft stets über variable sowie feste optische Parameter des operationsmikroskopischen Systems informiert. Die festen optischen Parameter charakterisieren dabei bevorzugt Komponenten des operationsmikroskopischen Systems, wie beispielsweise eingesetzte Linsen, Okulare oder dergleichen, und die variablen optischen Parameter charakterisieren dabei bevorzugt einstellbare Größen, wie beispielsweise ein Sichtfeld, eine Auflösung, eine Neigung der optischen Achse und dergleichen. Die Berücksichtigung der optischen Parameter des operationsmikroskopischen Systems ermöglicht vorteilhaft eine gleichzeitige Darstellung von OCT-Bildern und Videobilddaten mit optimaler Anpassung der Bilddaten aneinander sowie ebenfalls eine sequentielle Darstellung von OCT-Bildern und Videobilddaten mit optimalem Übergang.

In einer besonders bevorzugten Ausführungsform des Systems gemäß der vorliegenden Offenbarung weist das operationsmikroskopische System eine Stereokamera mit einem entlang einer ersten optischen Achse angeordneten und zum Erfassen eines ersten Bildsignals des Sichtfelds ausgebildeten ersten Stereoobjektiv (in Kombination mit einem ersten Bildsensor) und einem entlang einer zweiten optischen Achse angeordneten und zum Erfassen eines zweiten Bildsignals des Sichtfelds ausgebildeten zweiten Stereoobjektiv (in Kombination mit einem zweiten Bildsensor) auf. Das operationsmikroskopische System ist also zum Erfassen von stereoskopischen zeitaufgelösten Bildsignalen ausgebildet. Die erste optische Achse und die zweite optische Achse des operationsmikroskopischen Systems schließen dabei einen Winkel miteinander ein, wobei dieser Winkel zwischen den optischen Achsen gemeinsam mit dem Arbeitsabstand zwischen der Probe und den Objektiven den Stereowinkel der stereoskopischen Bilderfassung im operationsmikroskopischem System bestimmt. Gemäß dieser Ausführungsform ist die Steuereinheit bevorzugt dazu ausgebildet, den der Ermittlung des ersten OCT-Bilds und des zweiten OCT-Bilds zugrunde gelegten Stereowinkel entsprechend dem Stereowinkel des operationsmikroskopischen Systems zu bestimmen. Ferner ist die Steuereinheit bevorzugt dazu ausgebildet, anhand des ersten Bildsignals erste Videobilddaten und anhand des zweiten Bildsignals zweite Videobilddaten zu ermitteln. Diese Ausführungsform ermöglicht somit vorteilhaft eine stereoskopische Darstellung sowohl der Videobilddaten als auch der OCT-Bilder mit korrespondierendem Stereowinkel. Dies ermöglicht vorteilhaft eine stereoskopische gleichzeitige Darstellung mit minimalen Abweichungen als auch eine stereoskopische sequentielle Darstellung mit optimalem Übergang zwischen den verschiedenen Bildmodi (Video und OCT-Bilddaten). Gemäß dieser Ausführungsform sind oder umfassen die optischen Parameter des operationsmikroskopischen Systems zumindest die Parameter des operationsmikroskopischen Systems, die einen Stereowinkel desselben beeinflussen.

In einer ebenfalls bevorzugten Ausführungsform ist die Steuereinheit dazu ausgebildet, den Stereowinkel anhand einer Zoomstufe und/oder eines Arbeitsabstandes der Stereokamera zu ermitteln. Dabei ist der Abstand zwischen den Kameras der Stereokamera bevorzugt fest zueinander, so dass der Stereowinkel lediglich von dem Arbeitsabstand der Kameras zu der Probe abhängt. Dieser Arbeitsabstand ist bevorzugt anhand der Zoomstufe ermittelbar, beispielsweise in Kombination mit der Größe eines abgebildeten Sichtfelds oder eines Markers. Ist der Abstand der Kameras der Stereokamera zueinander variabel ausgebildet, beispielsweise bei per Kamerafahrt positionierbaren Kameras, wird bevorzugt auch der Abstand der Kameras zueinander bei der Ermittlung des Stereowinkels berücksichtigt. Gemäß dieser Ausführungsform ist die Steuereinheit bevorzugt dazu ausgebildet, den Stereowinkel des operationsmikroskopischen Systems zumindest anhand der Zoomstufe und/oder des Arbeitsabstands zu ermitteln und diesen Stereowinkel auch für die OCT-Bilder zu verwenden.

In einer ebenfalls bevorzugten Ausführungsform wird die Zoomstufe auch unabhängig vom Arbeitsabstand berücksichtigt. Beispielsweise ist bei einer Darstellung eines Sichtfelds mit dem operationsmikroskopischen System und kleinem Zoomwert die Verwendung eines kleinen Stereowinkels bevorzugt. Ein kleiner Zoomwert korrespondiert in der Regel zu einem großen Sehfeld, wobei eine Darstellung eines starken Tiefeneindrucks in der Regel nicht gewünscht ist. Bei einer Darstellung eines Sichtfelds mit dem operationsmikroskopischen System und großem Zoomwert ist die Verwendung eines größeren Stereowinkels bevorzugt. Ein großer Zoomwert korrespondiert zu einem kleinen Sehfeld, in dem eine Detaildarstellung mit starkem Tiefeneindruck häufig gewünscht ist. Somit wird bei hohen Vergrößerungen vorteilhaft auch ein Tiefeneindruck in der Darstellung der ermittelten OCT-Bilder erhöht.

In einer ferner bevorzugten Ausführungsform des Systems gemäß der vorliegenden Offenbarung weist dieses ferner ein medizintechnisches Instrument auf. Bei dem medizintechnischen Instrument handelt es sich beispielsweise um eine Sonde, einen Pointer, eine Pinzette, eine Ahle, einen Phako-Tip, ein Endoskop, eine Endo-LED oder dergleichen.

Gemäß dieser Ausführungsform ist die Steuereinheit ferner dazu eingerichtet, eine Position, einen Typ und/oder einen Zustand des medizintechnischen Instruments zu ermitteln. Ein Typ des medizintechnischen Instruments kann dabei bevorzugt anhand einer Geräteschnittstelle zum Anschluss des medizintechnischen Instruments, mittels eines Trackingsystems (beispielsweise durch Erkennen einer Target-ID) und/oder anhand einer Eingabe durch eine Nutzerschnittstelle erfolgen. Ebenso bevorzugt erfolgt die Erkennung eines Typs des in das Sichtfeld des operationsmikroskopischen Systems eingebrachten medizintechnischen Instruments durch Bildanalyse der Videobilddaten, beispielsweise mittels Segmentierung und Objekterkennung. Eine Position des medizintechnischen Instruments wird bevorzugt anhand der Detektion eines Markers und/oder eines mehrere Marker umfassendes Targets ermittelt, wobei der Marker eine Kennzeichnung auf oder eine Struktur des medizintechnischen Instruments sein kann. Die Erfassung des Markers und/oder des Targets erfolgt bevorzugt mittels des operationsmikroskopischen Systems und gegebenenfalls unter Verwendung zusätzlicher Lichtquellen (beispielsweise Infrarot-LEDs) und/oder nach einer Registrierung/Kalibrierung des medizintechnischen Instruments (beispielsweise durch Positionieren der einer Spitze des medizintechnischen Instruments an einem definierten Ort).

Ein Zustand des in das Sichtfeld des operationsmikroskopischen Systems eingebrachten medizintechnischen Instruments wird bevorzugt ebenfalls anhand von Bildanalyse der Videobilddaten ermittelt. So ist beispielsweise anhand der Bilddaten zu erkennen, ob eine Pinzette geöffnet oder geschlossen ist. Darüber hinaus kann eine Nutzereingabe zum Ändern eines Zustands von der Steuereinheit eingelesen werden, beispielsweise signalisiert eine Nutzereingabe zum Aktivieren eines Phako-Tips eine Änderung von dessen Zustand. Ferner kann ein an dem medizintechnischen Instrument angebrachter Sensor die Änderung von dessen Zustand detektieren, beispielsweise das Schließen einer Pinzette, und ein entsprechendes Sensorsignal an die Steuereinheit übermitteln.

Gemäß dieser bevorzugten Ausführungsform ist die Steuereinheit ferner dazu eingerichtet, einen Stereowinkel für das Ermitteln der stereoskopischen OCT-Bilder anhand der Position, des Typs und/oder des Zustands des medizintechnischen Instruments zu ermitteln. Ein Wechsel des Zustands eines medizintechnischen Instruments eines bestimmten Typs und/oder an einem bestimmten Ort ist bevorzugt indikativ für eine bestimmte Phase einer Operation. Mithin kann anhand der Erkennung von Position, des Typs und/oder des Zustands des medizintechnischen Instruments die für diese Phase optimale Darstellungsform gewählt werden. Dies umfasst neben der Wahl des Stereowinkels gegebenenfalls auch ein Wechsel von weiteren Darstellungsparametern der OCT-Bilddaten, beispielsweise einer Zoomstufe, einer virtuellen Blickrichtung, einer dargestellten Tiefe und/oder einer Schnittrichtung.

Anhand der vorgenannten Informationen ist die Steuereinheit beispielsweise in der Lage, automatisch oder basierend auf einer Nutzereingabe zu ermitteln, dass es sich bei einem durchgeführten Eingriff um ein Membranpeeling mittels einer Pinzette handelt und in diesem Fall ferner einen Abstand beziehungsweise eine Position der Pinzette relativ zu dem Auge zu ermitteln und in Abhängigkeit der vorgenannten Ermittlungen eine virtuelle Blickrichtung und/oder einen Stereowinkel der Darstellung der OCT-Bilder anzupassen beziehungsweise einzustellen. In einem weiteren Beispiel ist die Steuereinheit bevorzugt dazu in der Lage, automatisch oder basierend auf einer Nutzereingabe zu ermitteln, dass es sich bei einem durchgeführten Eingriff um eine subretinale Injektion eines "Bleb" oder um eine Platzierung eines Retina Stents handelt und basierend darauf eine virtuelle Blickrichtung und/oder einen Stereowinkel so anzupassen, dass ein Nutzer bei dem Positionieren der Nadel beziehungsweise des Stents optimal unterstütz wird, beispielsweise indem eine perspektivische Darstellung mit großem Tiefeneindruck (Stereowinkel) gewählt wird.

Die Funktionalitäten der erfindungsgemäßen Steuereinheit können durch elektrische oder elektronische Bauteile oder Komponenten (Hardware), durch Firmware (ASIC) implementiert sein und/oder durch Ausführen eines geeigneten Programms (Software) verwirklicht werden. Bevorzugt werden die Funktionalitäten der erfindungsgemäßen Steuereinheit durch eine Kombination von Hardware, Firmware und/oder Software verwirklicht, beziehungsweise implementiert. Beispielsweise sind einzelne Komponenten der erfindungsgemäßen Steuereinheit zum Ausführen einzelner Funktionalitäten als separat integrierter Schaltkreis ausgebildet oder auf einem gemeinsamen integrierten Schaltkreis angeordnet.

Die einzelnen Funktionalitäten der erfindungsgemäßen Steuereinheit sind ferner bevorzugt als ein oder mehrere Prozesse ausgebildet, die auf einem oder mehreren Prozessoren in einem oder mehreren elektronischen Rechengeräten laufen und beim Ausführen von ein oder mehreren Computerprogrammen erzeugt werden. Die Steuereinheit ist dabei dazu ausgebildet, mit den anderen Komponenten, insbesondere der Nutzerschnittstelle, dem OCT-System und dem Anzeigemittel zusammenzuarbeiten, um die hierin beschriebenen Funktionalitäten des erfindungsgemäßen Systems zu verwirklichen. Dem Fachmann ist ferner ersichtlich, dass die Funktionalitäten von mehreren Computern (Datenverarbeitungsgeräten, Steuereinheiten, Steuergeräte) kombiniert oder in einem einzigen Gerät kombiniert sein können oder dass die Funktionalität von einem bestimmten Datenverarbeitungsgerät auf eine Vielzahl von Geräten verteilt vorliegen kann, um die Funktionalitäten der erfindungsgemäßen Steuereinheit zu realisieren.

In einer besonders bevorzugten Ausführungsform des erfindungsgemäßen Systems ist dieses in ein Operationsmikroskop integriert. Dabei weist das Operationsmikroskop bevorzugt ein OCT-System wie obenstehend beschrieben auf. Ferner bevorzugt weist das Operationsmikroskop eine Schnittstelle für eine Nutzereingabe und ein Anzeigemittel auf oder ist es mit diesen verbunden. Bevorzugt weist das Operationsmikroskop ferner ein operationsmikroskopisches System wie obenstehend beschrieben auf, wobei Bildsensor und Optik Teil einer Kamera, insbesondere einer Hauptbeobachterkamera oder einer Umfeldkamera des Operationsmikroskops, sind. Die Steuereinheit des Operationsmikroskops ist bevorzugt als Steuereinheit des erfindungsgemäßen Systems ausgebildet und ist insbesondere dazu ausgebildet, anhand von auf einer Speichereinheit des Operationsmikroskops gespeicherten Befehlen das erfindungsgemäße Verfahren wie nachfolgend beschrieben durchzuführen.

Unter einem Operationsmikroskop wird im Rahmen der vorliegenden Offenbarung im weitesten Sinne ein für den Einsatz während einer Operation geeignetes Mikroskop verstanden. Das Operationsmikroskop weist bevorzug eine Halterung auf, die eine Abbildung des Operationsgebiets unabhängig von Kopfbewegungen des Operateurs erlaubt. Ferner bevorzugt weist das Operationsmikroskop zumindest einen Strahlteiler und zumindest zwei Okulare auf. Alternativ handelt es sich bei dem Operationsmikroskop um ein reines "Digiskop" ohne Okulare. Ebenfalls bevorzugt weist das Operationsmikroskop zumindest einen bildgebenden Sensor auf. Ferner bevorzugt weist das Operationsmikroskop eine Hauptbeobachterkamera und eine Umfeldkamera auf. Das Operationsmikroskop kann kinematische beziehungsweise robotische Hilfsmittel zum Durchführen operativer Eingriffe aufweisen. Alternativ kann ein Operationsmikroskop als medizintechnisches Mikroskop, medizinisch zugelassenes Mikroskop oder medizinisches Mikroskop bezeichnet werden.

Ein weiterer Aspekt der vorliegenden Offenbarung betrifft ein Verfahren zum Erfassen und Visualisieren von OCT-Signalen. Dieses weist den Verfahrensschritt des Erfassens eines zeitaufgelösten OCT-Signals von einem ausgewählten Sichtfeld einer Probe mittels eines OCT Systems (wie obenstehend beschrieben) auf. Dabei weist das OCT-Signal eine Vielzahl von Tupeln auf, die jeweils ein Volumenelement der Probe und eine zu dem Volumenelement korrespondierende Streuintensität aufweisen beziehungsweise repräsentieren. Das Verfahren weist ferner die Schritte des Ermittelns einer vorgegebenen virtuellen Blickrichtung und des Ermittelns eines zeitaufgelösten OCT-Bilds anhand der Tupel, einer Auflösung eines Anzeigemittels und der virtuellen Blickrichtung als dreidimensionales und aus der virtuellen Blickrichtung perspektivisches Volumenbild auf. Im Verfahren gemäß der vorliegenden Offenbarung erfolgt schließlich das Darstellen des zeitaufgelösten OCT-Bilds auf dem Anzeigemittel, als aus der virtuellen Blickrichtung perspektivisches Volumenbild. Das Verfahren gemäß vorliegender Offenbarung realisiert dieselben Vorteile wie das System gemäß vorliegender Offenbarung und diesbezüglich wird auf obige Ausführungen verwiesen.

In einer bevorzugten Durchführungsform des Verfahrens gemäß der vorliegenden Offenbarung weist dieses ferner den Schritt des Ermittelns eines zeitaufgelösten ersten OCT-Bilds und eines zeitaufgelösten zweiten OCT-Bilds auf. Dabei werden das erste und das zweite OCT-Bild jeweils anhand der Tupel, anhand der Auflösung des Anzeigemittels, anhand der der Blickrichtung und zusätzlich anhand eines Stereowinkels ermittelt und zwar jeweils als dreidimensionales und aus der virtuellen Blickrichtung perspektivisches Volumenbild. Gemäß dieser Durchführungsform erfolgt ferner ein stereoskopisches Darstellen des zeitaufgelösten ersten OCT-Bilds und des zeitaufgelösten zweiten OCT-Bilds auf dem Anzeigemittel. Dies ermöglicht vorteilhaft die stereoskopische Darstellung mit variabler virtueller Blickrichtung und variablem Stereowinkel.

In einer ferner bevorzugten Durchführungsform des Verfahrens gemäß der vorliegenden Offenbarung weist dieses ferner den Schritt des Ermittelns einer vorgegebenen virtuellen Blickrichtung, basierend auf einer mittels einer Schnittstelle erfassten Nutzereingabe und/oder basierend auf einem mittels einer Geräteschnittstelle erfassten Geräteparameter, auf.

Weitere bevorzugte Durchführungsformen des Verfahrens gemäß der vorliegenden Offenbarung korrespondieren zur weiteren bevorzugten Ausführungsformen des Systems gemäß der vorliegenden Offenbarung und realisieren dieselben Vorteile wie die Ausführungsformen.

Ein weiterer Aspekt der vorliegenden Offenbarung betrifft ein Computerprogramm, umfassend Befehle, die bei der Ausführung durch eine Steuereinheit wie obenstehend beschrieben, bevorzugt eines Operationsmikroskops wie obenstehend beschrieben, bewirken, dass das System oder Operationsmikroskop wie obenstehend beschrieben das erfindungsgemäße Verfahren wie obenstehend beschrieben ausführen. Das Computerprogramm umfasst bevorzugt Befehle, die bei der Ausführung durch eine Steuereinheit wie obenstehend beschrieben, bevorzugt eines Operationsmikroskops, bewirken, dass das System oder Operationsmikroskop wie obenstehend beschrieben, das erfindungsgemäße Verfahren gemäß einer der bevorzugten Durchführungsformen wie obenstehend beschrieben ausführen. Das erfindungsgemäße Computerprogramm ist dabei bevorzugt in einem flüchtigen Speicher, beispielsweise einem RAM-Element, oder in einem nichtflüchtigen Speichermedium, wie beispielsweise einer CD-ROM, einem Flash-Speicher oder dergleichen, abgelegt.

Weitere bevorzugte Ausgestaltungen der Erfindung ergeben sich aus den übrigen, in den Unteransprüchen genannten Merkmalen und den nachfolgend erläuterten Figuren. Die verschiedenen in dieser Anmeldung genannten Ausführungsformen der Erfindung sind, sofern im Einzelfall nicht anders ausgeführt, mit Vorteil miteinander kombinierbar.

### Beschreibung der Figuren

Die Erfindung wird nachfolgend in Ausführungsbeispielen anhand der zugehörigen Zeichnungen erläutert. Es zeigen:
- Figur 1: eine schematische Darstellung eines Systems gemäß einer ersten Ausführungsform;
- Figur 2: eine schematische Darstellung eines Systems gemäß einer zweiten Ausführungsform;
- Figur 3: eine schematische Darstellung einer virtuellen Blickrichtung;
- Figur 4: eine schematische Darstellung einer Visualisierung von OCT-Signalen als OCT-Bilder in Abhängigkeit einer virtuellen Blickrichtung;
- Figur 5: eine schematische Darstellung einer Probe und eines Anzeigemittels gemäß einer Ausführungsform;
- Figur 6: eine schematische Darstellung eines Anzeigemittels und eines medizintechnischen Instruments gemäß einer weiteren Ausführungsform; und
- Figur 7: ein schematisches Ablaufdiagram eines Verfahrens gemäß einer Durchführungsform.

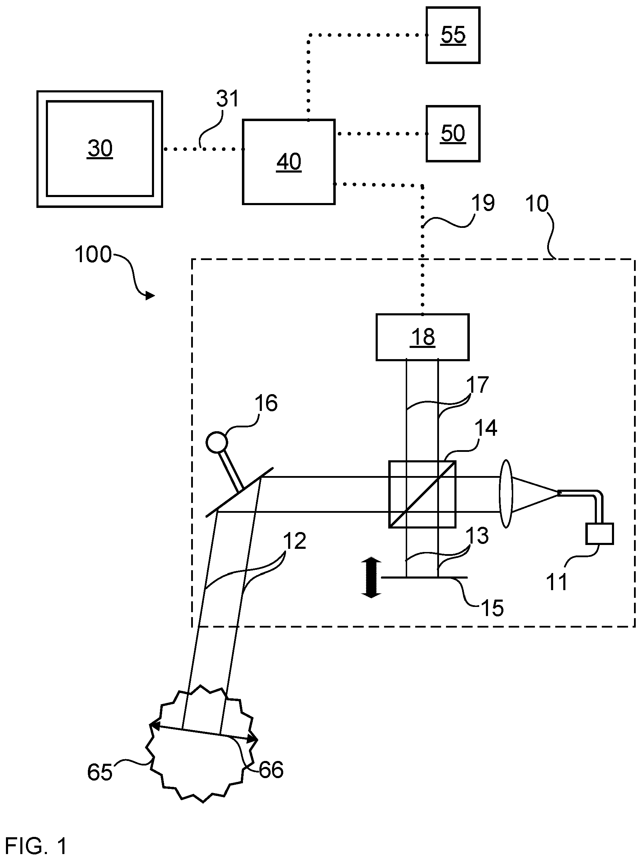

Figur 1 zeigt eine schematische Darstellung eines Systems 100 zum Erfassen und Visualisieren von OCT-Signalen 19 gemäß einer ersten Ausführungsform.

Das System 100 weist ein OCT-System 10 mit einer breitbandigen Lichtquelle 11, beispielsweise einer Superlumineszenzdiode, auf. Das Licht der Lichtquelle 11 wird in ein Interferometer aufweisend einen beweglichen Spiegel 15 und einen Strahlteiler 14, geleitet. Im Strahlteiler 14 wird das Licht in einen Probenstrahl 12 und in einen Referenzstrahl 13 geteilt. Der Probenstrahl 12 wird mittels eines Scanspiegels 16 über die Probe 65 gerastert, wobei zumindest ein Abschnitt eines gewählten Sichtfelds 66 der Probe 65 abgerastert wird. Der Referenzstrahl 13 wird auf den beweglichen Spiegel 15 gelenkt und von diesem zurück auf den Strahlteiler 14 reflektiert. Der Probenstrahl 12 interagiert mit der Probe 65, insbesondere dem Volumen der Probe 65 und wird von diesem zu dem Scanspiegel 16 zurückgestreut und von diesem auf den Strahlteiler 14 gelenkt. Dort werden der zurückgestreute Probenstrahl 12 und der reflektierte Referenzstrahl 13 zur Überlagerung gebracht, wobei ein Gangunterschied zwischen den überlagerten Strahlen 12, 13 durch den beweglichen Spiegel 15 eingestellt wird. Das so erzeugte Interferenzmuster 17 wird mittels eines Detektors 18, beispielsweise eines CCD-Detektors oder eines CMOS-Detektors, erfasst.

Das so erfasste zeitaufgelöste OCT-Signal 19 wird von dem Detektor 18 zur Steuereinheit 40 übermittelt. Die Steuereinheit 40 empfängt ferner eine Nutzereingabe zu einer gewünschten virtuellen Blickrichtung mittels einer Nutzerschnittstelle 50, wobei es sich bevorzugt um ein HMD mit Mitteln zum Erfassen einer Kopf- und/oder Augenbewegung eines Nutzers handelt. Alternativ oder zusätzlich empfängt die Steuereinheit 40 einen Geräteparameter eines über eine Geräteschnittstelle 55 angeschlossenen Geräts, beispielsweise eines operationsmikroskopischen Systems 20, eines anderen bildgebenden Systems oder eines medizintechnischen Instruments 70. Ferner kann der Geräteparameter von einem an die Geräteschnittstelle 55 angeschlossenen Trackingsystem (nicht dargestellt) stammen aber ein medizintechnisches Instrument charakterisieren, beispielsweise dessen Raumlage.

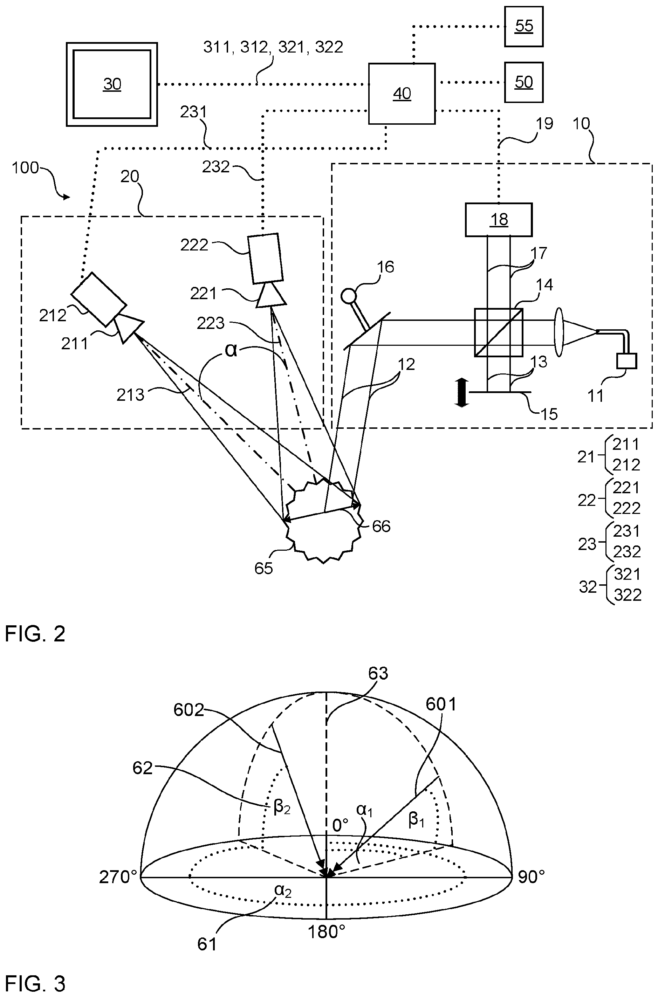

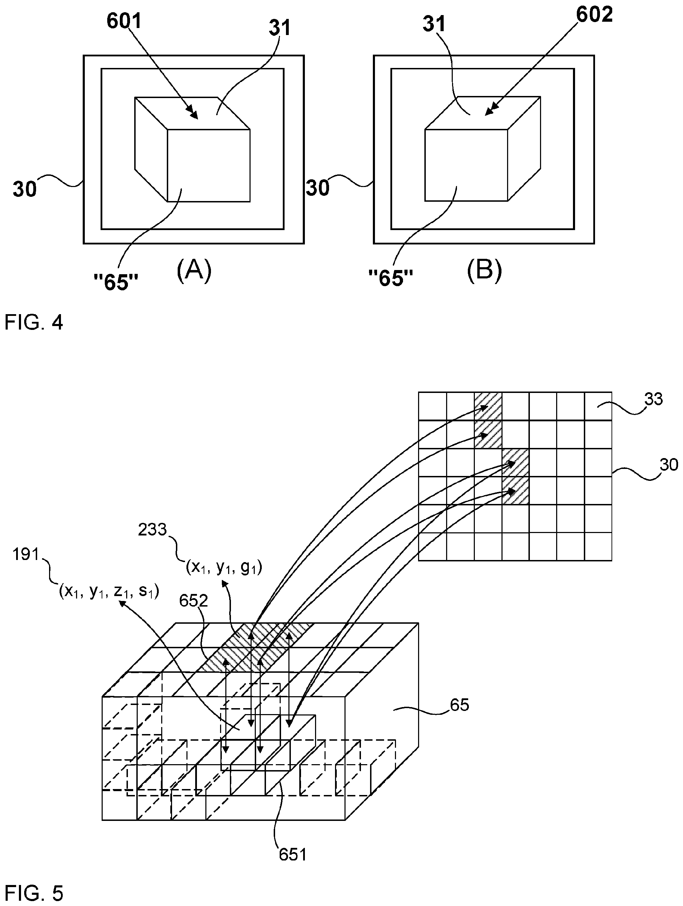

Die Steuereinheit 40 ermittelt zu dem erfassten zeitaufgelösten OCT-Signal 19 und basierend auf einer vorgegebenen virtuellen Blickrichtung 60 ein zeitaufgelöstes OCT-Bild 31 und übermittelt das zeitaufgelöste OCT-Bild 31 zur Darstellung an das Anzeigemittel 30. Die virtuelle Blickrichtung 60 ist dabei durch die Steuereinheit 40 anhand einer Nutzereingabe in Nutzerschnittstelle 50 und/oder basierend auf einem mittels der Geräteschnittstelle 55 ermittelten Geräteparameter bestimmt. Zur Erläuterung bezüglich der virtuellen Blickrichtung 60 wird auf die nachfolgende Beschreibung der Figuren 3 und 4 verwiesen.

Figur 2 zeigt eine schematische Darstellung eines Systems 100 zum Erfassen und Visualisieren von OCT-Signalen 19 gemäß einer zweiten Ausführungsform. Gleiche Komponenten sind mit gleichen Bezugszeichen wie in Figur 1 bezeichnet und auf eine wiederholte Beschreibung dieser Komponenten wird aus Gründen der Knappheit verzichtet.

Das System 100 der Figur 2 unterscheidet sich von dem der Figur 1 dadurch, dass es ferner ein operationsmikroskopisches System 20 mit einer ersten Kamera 21 mit einer ersten Optik 211 und einem ersten Bildsensor 212 und eine zweite Kamera 22 mit einer zweiten Optik 221 und einem zweiten Bildsensor 222 aufweist. Mit jeder dieser Kameras 21, 22 ist das Sichtfeld 66 der Probe 65 entlang einer optischen Achse erfassbar, so dass im Ergebnis ein stereoskopisches Bildsignal 23 erfasst wird, welches ein erstes Bildsignal 231 und ein zweites Bildsignal 232 aufweist. Bei den Kameras 21, 22 handelt es sich beispielsweise um die beiden Kameras einer als Stereokamera ausgebildeten Hauptbeobachterkamera eines Operationsmikroskops.

Gemäß der Ausführungsform der Figur 2 ermittelt die Steuereinheit 40 zu dem erfassten stereoskopischen Bildsignal 23 korrespondierende stereoskopische Videobilddaten 32, wobei ein Stereowinkel α zwischen den optischen Achsen 213, 223 der beiden Kameras 21, 22 einen Tiefeneindruck beziehungsweise eine Tiefenwahrnehmung der anhand des stereoskopischen Bildsignals 23 erstellten stereoskopischen Videobilddaten 32 bestimmt. Die stereoskopischen Videobilddaten 32 weisen dabei zu dem ersten Bildsignal 231 korrespondierende erste Videobilddaten 321 und zu dem zweiten Bildsignal 232 korrespondierende zweite Videobilddaten 322 auf. Der Stereowinkel α hängt neben dem Abstand der Kameras 21, 22 zueinander von einem Arbeitsabstand zwischen den Kameras 21, 22 und der Probe 65 ab.

Ein zeitaufgelöstes OCT-Signal 17 wird in dem System 100 der Figur 2 in gleicher Weise erfasst, wie mit Bezug zu Figur 1 bereits beschrieben. Die Steuereinheit 40 ermittelt jedoch anhand des OCT-Signals 17 ein zu den ersten Videobilddaten 321 korrespondierendes erstes OCT-Bild 311 und ein zu den zweiten Videobilddaten 321 korrespondierendes zweites OCT-Bild 312. Das erste OCT-Bild 311 und das zweite OCT-Bild 312 werden dabei beispielsweise so erzeugt, als wären diese unter demselben Stereowinkel α erfasst worden, wie das erste und zweite Bildsignal 231, 232. Ebenfalls kann ein Stereowinkel der OCT-Bilder 311, 312 jedoch auch anhand einer mittels der Schnittstelle 50 erfassten Nutzereingabe abweichend eingestellt werden. Die ersten und zweiten Videobilddaten 321, 322 werden gemeinsam mit den ersten und zweiten OCT-Bildern 311, 312 gleichzeitig oder sequentiell auf dem Anzeigemittel 30 dargestellt, wobei ein Wechsel zwischen den OCT-Bildern 311, 312 und den Videobilddaten 321, 322 beispielsweise anhand einer Eingabe mittels Schnittstelle 50 erfolgt.

Figur 3 zeigt eine schematische Darstellung einer virtuellen Blickrichtung 60, insbesondere einer ersten virtuellen Blickrichtung 601 und einer zweiten virtuellen Blickrichtung 602. Jede dieser virtuellen Blickrichtungen 601, 602 ist dabei durch einen Azimuthwinkel 61 und durch einen Höhenwinkel 62 relativ zu einer optischen Achse 63 des OCT-Systems 10 definiert. Die optische Achse des OCT-Systems 10 steht dabei bevorzugt senkrecht auf einer Oberfläche der Probe 65. Der Azimuthwinkel 61 ist ein ebener Winkel zwischen 0° und 360° in einer zu der optischen Achse 63 senkrechten Ebene. Der Höhenwinkel 62 ist ein ebener Winkel zwischen 0° und 90° in einer die optische Achse 63 enthaltenden Ebene. Der Höhenwinkel ist ein ebener Winkel und wird zwischen der virtuellen Blickrichtung 601, 602 und der horizontalen (Proben)Ebene gemessen. In der Darstellung von Figur 3 ist eine erste virtuellen Blickrichtung 601 durch einen ersten Azimuthwinkel α₁ von etwa 45°und durch einen ersten Höhenwinkel β₁ von etwa 30° definiert und ist eine zweite virtuellen Blickrichtung 602 durch einen zweiten Azimuthwinkel α₂ von etwa 315° und durch einen zweiten Höhenwinkel β₂ von etwa 60° definiert.

Figur 4 zeigt eine schematische Darstellung einer Visualisierung von OCT-Signalen 19 als OCT-Bilder 31 in Abhängigkeit einer virtuellen Blickrichtung 60. Figur 4(A) zeigt dabei ein OCT-Bild 31 der Probe 65 (Bild der Probe "65") aus einer ersten virtuellen Blickrichtung 601 mit dem ersten Azimuthwinkel α₁ und Figur 4(B) zeigt dabei ein abweichendes OCT-Bild 31 der Probe 65 (Bild der Probe "65") aus einer zweiten virtuellen Blickrichtung 602 mit dem zweiten Azimuthwinkel α₂. Auf eine Darstellung der Auswirkung der verschiedenen Höhenwinkel β₁ und β₂ wurde aus Gründen der Übersichtlichkeit verzichtet.

Figur 5 zeigt eine schematische Darstellung einer Probe 65 und eines Anzeigemittels 30 gemäß einer Ausführungsform. Die Probe 65 weist dabei eine Vielzahl von Volumenelementen 651 und eine Vielzahl von Oberflächenelementen 652 auf, wobei bestimmte Volumenelemente 651 zu bestimmten Oberflächenelementen 652 korrespondieren. Eine beispielhafte Auswahl von Oberflächenelementen 652 ist schraffiert dargestellt und während die Mehrheit der Volumenelemente 651 mit gestrichenen Linien dargestellt sind, sind die vier zu den schraffierten Oberflächenelementen 652 korrespondierenden Volumenelemente 152 mit durchgezogenen Linien dargestellt. Ferner verbinden doppelseitige Pfeile diese Volumenelemente 651 mit den zugehörigen Oberflächenelementen 652.

Mit dem OCT-System 10 des Systems 100 der Figur 1 ist insbesondere das Volumen der Probe 65 erfassbar, indem von dieser gestreutes kurzwelliges Licht des Probenstrahls 12 über den Scanspiegel 16 mittels des Interferometers mit dem Referenzstrahl 17 überlagert wird. Das so erzeugte und mittels dem Detektor 18 als zeitaufgelöstes OCT-Signal 19 erfasste Interferenzmuster 17 weist eine Vielzahl von ersten Tupeln 191 auf, wobei sich eine Anzahl der ersten Tupel 191 beispielsweise aus einer Anzahl der mit dem Scanspiegel 16 abgerasterten Punkte auf der Probe 65 ergibt. Jedes der ersten Tupel 191 korrespondiert dabei zu einem der dargestellten Volumenelemente 651 und weist einen Wert einer Streuintensität *sᵢ* auf. Anhand einer Kalibrierung beziehungsweise Registrierung des OCT-Systems 10 relativ zu einem Koordinatensystem der Probe 65 (Patient) sind jedem der ersten Tupel 191 ferner drei Raumkoordinaten *xᵢ, yᵢ, zᵢ* zugeordnet. In dem dargestellten Beispiel weist ein erstes Tupel 191 die Raumkoordinaten *x₁, y₁, z₁* und den Streuintensitätswert *s₁* auf.

Mit dem operationsmikroskopischen System 20 des Systems 100 der Figur 2 ist insbesondere die Oberfläche der Probe 65 erfassbar, indem von dieser reflektiertes beziehungsweise rückgestreutes langwelliges (sichtbares) Licht über die Optiken 211, 221 auf die Bildsensoren 212, 222 der Kameras 21, 22 zurückgeworfen wird. Die mittels der Bildsensoren 212, 222 erfassten Bildsignale 321, 322 weisen dann jeweils eine Vielzahl von zweiten Tupeln 233 auf, wobei sich eine Anzahl der zweiten Tupel 233 jeweils aus einer Auflösung des zugehörigen Bildsensors 212, 222 ergibt. Jedes der zweiten Tupel 233 korrespondiert dabei zu einem der dargestellten Oberflächenelemente 652 und weist einen Wert einer Graustufe *gᵢ* auf, der eine Intensität des auf den jeweiligen Bildsensor 212, 222 zurückgeworfenen Lichts entspricht. Anhand einer Kalibrierung beziehungsweise Registrierung der Bildsensoren 212, 222 relativ zu einem Koordinatensystem der Probe 65 (Patient) sind jedem der zweiten Tupel 233 ferner zwei laterale Raumkoordinaten *xᵢ, yᵢ* zugeordnet. In dem dargestellten Beispiel weist ein zweites Tupel 233 die lateralen Raumkoordinaten *x₁, y₁* und den Graustufenwert *g₁* auf.

Das in Figur 5 ferner dargestellte Anzeigemittel 30 weist eine Vielzahl von Pixeln 33 auf, insbesondere 42 Pixel mit 7 Pixeln in horizontaler Richtung und 6 Pixeln in vertikaler Richtung. In dem dargestellten Beispiel ergab sich aus der Auflösung des Bildsensors 12 eine Erfassung der Probenoberfläche durch die Bildsignale 23 in 21 Oberflächenelementen 652 mit 7 Oberflächenelementen 652 in horizontaler Richtung und 3 Oberflächenelementen 652 in vertikaler Richtung. Somit korrespondiert in den Videobilddaten 32 in horizontaler Richtung ein Pixel 33 zu einem Oberflächenelement 652 und korrespondieren in vertikaler Richtung zwei Pixel 33 zu einem Oberflächenelement 652. Zu schraffiert dargestellten Oberflächenelementen 652 korrespondierende Pixel 33 sind ebenfalls schraffiert dargestellt und die Zuordnung ist ferner durch Pfeile verdeutlicht. Wie ferner durch Pfeile dargestellt, wird durch die Steuereinheit 40 des Systems 100 auch das zugehörige OCT-Bild 31 der zu den Oberflächenelementen 652 korrespondierenden Volumenelementen 651 so erzeugt und in den jeweiligen Pixeln 33 dargestellt, dass Videobilddaten 32 von bestimmten Oberflächenelementen 652 auf bestimmten Pixeln 33 dargestellt werden und OCT-Bilder 31 von zu den Oberflächenelementen 652 korrespondierenden Volumenelementen 651 ebenfalls auf den bestimmten Pixeln 33 dargestellt werden. Mithin werden korrespondierende OCT-Bilder 31 und Videobilddaten 31 am selben Ort des Anzeigemittels 30 dargestellt.

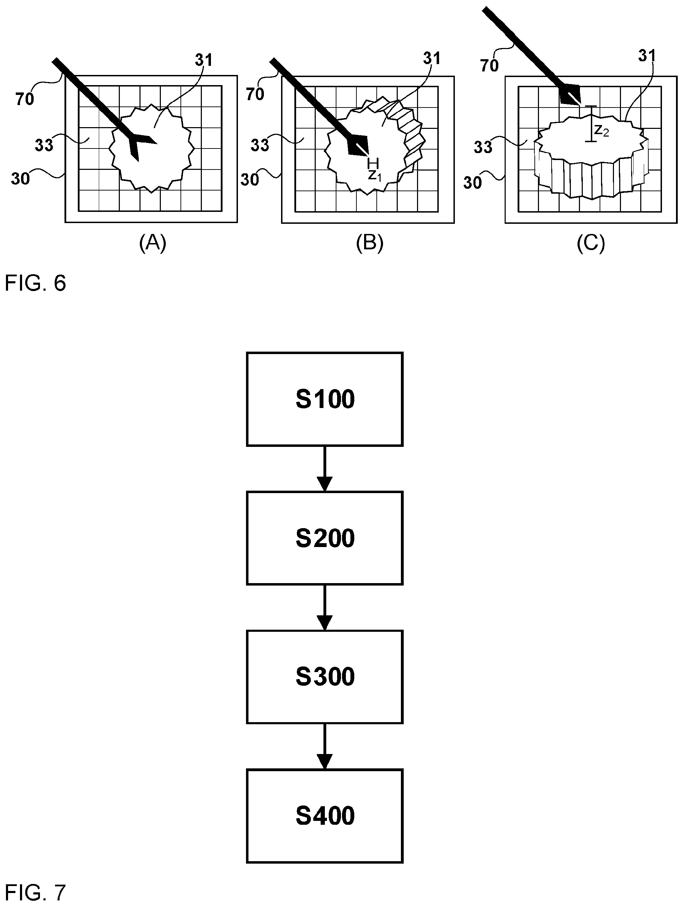

Eine schematische Darstellung eines Anzeigemittels 30 gemäß einer weiteren Ausführungsform ist in Figur 6 dargestellt. Dabei weist das System 100 wie in Figur 2 dargestellt ferner ein medizintechnisches Instrument 70, insbesondere eine Pinzette 70, auf. In einer in Figur 6(A) dargestellten Annäherungsphase der Pinzette 70 an die Probe 15 erfolgt eine Darstellung der geöffneten Pinzette 70 und der Probe 15 auf dem Anzeigemittel 30 in Form eines OCT-Bilds 31 in *"en face"* Ansicht (Draufsicht), also mit einem, wie in Figur 3 dargestellten, Höhenwinkel von 90°. Sobald durch die Steuereinheit 40 anhand von Sensorwerten und/oder anhand der einer Auswertung der OCT-Bilder 31 oder gegebenenfalls von Videobilddaten 32 erkannt wird, dass die Pinzette 70 geschlossen ist und ein vertikaler Abstand zwischen einer Spitze der Pinzette 70 und einer Oberfläche der Probe 65 gleich einem vorbestimmten Grenzwert Δz₁ oder geringer ist, wechselt die Steuereinheit 40 die Darstellung von Probe 65 und gegebenenfalls von Pinzette 70 auf dem Anzeigemittel 30 mittels des OCT-Bilds 31 von der *"en face"* Ansicht (Draufsicht) in eine andere virtuelle Blickrichtung 60. Insbesondere ermittelt die Steuereinheit 40 ein OCT-Bild 31 wie in Figur 6(B) dargestellt, nämlich eine perspektivische Volumendarstellung mit einem Höhenwinkel von etwa 45°. In dem dargestellten Beispiel wird die Pinzette 70 genutzt, um einen Teil der Oberfläche der Probe 65 an- beziehungsweise abzuheben. Sobald durch die Steuereinheit 40 anhand von Sensorwerten, des OCT-Bildes 31 und/oder der Videobilddaten 32 erkannt wird, dass die Pinzette 70 geschlossen ist und ein vertikaler Abstand zwischen einer Spitze der Pinzette 70 und einer Oberfläche der Probe 65 außerhalb der anbeziehungsweise abgehobenen Oberfläche der Probe 65 (beispielsweise um einem Mindestabstand lateral beabstandet) gleich einem vorbestimmten Grenzwert Δz₂ oder größer ist, wechselt die Steuereinheit 40 die virtuelle Blickrichtung 60 der Darstellung des OCT-Bildes 32 erneut. Wie in Figur 6(C) dargestellt, wird dieses nun in einer seitlichen Perspektive, sprich mit einem Höhenwinkel von nahe 0° auf dem Anzeigemittel 30 gezeigt. Somit wird eine Interaktion von Operateur und Operationsmikroskop durch die vorteilhafte Darstellung des erfassten OCT-Signals 19 kontinuierlich und objektiv verbessert, indem die virtuelle Blickrichtung 60 der Darstellung anhand einer mittels der Schnittstelle 50 erfassten Nutzereingabe verbessert wird.

Figur 7 zeigt ein schematisches Ablaufdiagram eines Verfahrens gemäß einer Durchführungsform. Das Verfahren weist einen ersten Schritt S100 des Erfassens eines zeitaufgelösten OCT-Signals 19 eines ausgewählten Sichtfelds 66 einer Probe 65 mittels eines OCT-Systems 10 auf, wobei das OCT-Signal 19 eine Vielzahl von Tupeln 191 aufweist, die jeweils ein Volumenelement 651 der Probe 65 und eine zu dem Volumenelement 651 korrespondierende Streuintensität repräsentieren. Das Verfahren weist ferner den zweiten Schritt S200 des Ermittelns einer vorgegebenen virtuellen Blickrichtung 60 auf. In einem dritten Schritt S300 erfolgt ein Ermitteln eines zeitaufgelösten OCT-Bilds 31 anhand der Tupel 191, einer Auflösung eines Anzeigemittels 30 und der virtuellen Blickrichtung 60 als ein dreidimensionales und aus der virtuellen Blickrichtung 60 perspektivisches Volumenbild. Schließlich erfolgt in einem vierten Schritt S400 das Darstellen des zeitaufgelösten OCT-Bilds 31 auf dem Anzeigemittel 30.

### Bezugszeichenliste

- 10: OCT-System
- 11: breitbandige Lichtquelle
- 12: Probenstrahl
- 13: Referenzstrahl
- 14: Strahlteiler (Interferometer)
- 15: beweglicher Spiegel (Interferometer)
- 16: Scanmechanismus (Scanspiegel)
- 17: Interferenzmuster
- 18: Detektor
- 19: zeitaufgelöstes OCT-Signal
- 191: Tupel (OCT)
- 20: operationsmikroskopisches System
- 21: erste Kamera
- 211: erste Optik
- 212: erster Bildsensor
- 213: erste optische Achse
- 22: zweite Kamera
- 221: zweite Optik
- 222: zweiter Bildsensor
- 223: zweite optische Achse
- 23: zeitaufgelöstes Bildsignal
- 231: erstes Bildsignal
- 232: zweites Bildsignal
- 233: Tupel (Bildsignal)
- 30: Anzeigemittel
- 31: OCT-Bild
- 311: erstes OCT-Bild
- 312: zweites OCT-Bild
- 32: Videobilddaten
- 321: erste Videobilddaten
- 322: zweite Videobilddaten
- 33: Pixel
- 40: Steuereinheit
- 50: Nutzerschnittstelle
- 55: Geräteschnittstelle
- 60: virtuelle Blickrichtung
- 601: erste virtuelle Blickrichtung
- 602: zweite virtuelle Blickrichtung
- 61: Azimuthwinkel
- 62: Höhenwinkel
- 63: optische Achse des OCT Systems
- 65: Probe
- 66: Sichtfeld
- 651: Oberflächenelement
- 652: Volumenelement
- 70: medizintechnisches Instrument

## Patentansprüche

1. System (100) zum Erfassen und Visualisieren von OCT-Signalen, aufweisend:
ein OCT-System (10);
ein zur zeitaufgelösten Anzeige von Bilddaten (31) als stereoskopische Bilder ausgebildetes Anzeigemittel (30); und
eine Steuereinheit (40), die dazu eingerichtet ist, das OCT-System (10) zum Erfassen eines zeitaufgelösten OCT-Signals (19) eines ausgewählten Sichtfelds (66) der Probe (65) anzusteuern und anhand des erfassten zeitaufgelösten OCT-Signals (19), einer vorgebbaren virtuellen Blickrichtung (60) und eines Stereowinkels α ein zeitaufgelöstes erstes OCT-Bild (311) und ein zeitaufgelöstes zweites OCT-Bild (312) zu ermitteln und das erste zeitaufgelöste OCT-Bild (311) und das zweite zeitaufgelöste OCT-Bild (312) auf dem Anzeigemittel (30) stereoskopisch darzustellen,
wobei die Steuereinheit (40) ferner dazu eingerichtet ist, eine Phase einer durchgeführten Operation zu ermitteln und den Stereowinkel α und/oder die virtuelle Blickrichtung anhand der ermittelten Phase der durchgeführten Operation zu ermitteln.

2. System (100) nach Anspruch 1, wobei die virtuelle Blickrichtung (60) durch einen Azimuthwinkel (61) und einen Höhenwinkel (62) relativ zur optischen Achse (63) des OCT-Systems (10) definiert ist.

3. System (100) nach Anspruch 1 oder 2, wobei die Steuereinheit (40) dazu eingerichtet ist, das zeitaufgelöste OCT-Bild (31) als dreidimensionales und aus der virtuellen Blickrichtung (60) perspektivisches Volumenbild zu ermitteln.

4. System nach einem der vorangehenden Ansprüche, wobei das System (100) ferner eine zum Erfassen einer Nutzereingabe ausgebildete Schnittstelle (50) aufweist und die virtuelle Blickrichtung basierend auf einer Nutzereingabe bestimmt wird und/oder wobei das System (100) ferner eine zum Erfassen eines Geräteparameters ausgebildete Geräteschnittstelle (55) aufweist und die virtuelle Blickrichtung basierend auf einem mittels der Geräteschnittstelle (55) erfassten Geräteparameter bestimmt wird.

5. System (100) nach einem der vorangehenden Ansprüche, wobei das OCT-Signal (19) eine Vielzahl von Tupeln (191) aufweist, die jeweils ein Volumenelement (651) der Probe (65) und eine Streuintensität repräsentieren, wobei das Anzeigemittel (30) eine Vielzahl von Pixeln (33) aufweist und wobei die Steuereinheit (40) dazu eingerichtet ist, OCT-Bilder (31) anhand der Tupel (191), einer Auflösung des Anzeigemittels (30) und der virtuellen Blickrichtung (60) so zu ermitteln, dass bestimmte Pixel (33) zu bestimmten Volumenelementen (651) korrespondieren.

6. System (100) nach einem der vorangehenden Ansprüche, ferner aufweisend:
ein zum Erfassen eines zeitaufgelösten Bildsignals (23) des ausgewählten Sichtfelds (66) der Probe (65) ausgebildetes operationsmikroskopisches System (20),
wobei die Steuereinheit (40) ferner dazu eingerichtet ist, anhand des erfassten zeitaufgelösten Bildsignals (23) korrespondierende Videobilddaten (32) zu ermitteln und die Videobilddaten (32) gleichzeitig oder sequentiell mit dem zeitaufgelösten OCT-Bild (31) auf dem Anzeigemittel (30) darzustellen.

7. System (100) nach Anspruch 6, wobei die Steuereinheit (40) dazu eingerichtet ist, die Videobilddaten (32) und das zeitaufgelöste OCT-Bild (31) sequentiell mit gleicher Vergrößerung, gleicher Perspektive und/oder gleichem Stereowinkel α auf dem Anzeigemittel (30) darzustellen.

8. System (100) nach Anspruch 6, wobei die Steuereinheit (40) dazu eingerichtet ist, die Videobilddaten (32) mit einer ersten Transparenzstufe und das zeitaufgelöste OCT-Bild (31) mit einer zweiten Transparenzstufe gleichzeitig auf dem Anzeigemittel (30) darzustellen.

9. System (100) nach Anspruch 1, wobei die Steuereinheit (40) ferner dazu eingerichtet ist, den Stereowinkel α basierend auf einer mittels der Schnittstelle (50) erfassten Nutzereingabe zu ermitteln.

10. System (100) nach Anspruch 6, wobei die Steuereinheit (40) ferner dazu eingerichtet ist, den Stereowinkel α anhand optischer Parameter des operationsmikroskopischen Systems (20) zu ermitteln.

11. System (100) nach Anspruch 6, wobei das operationsmikroskopische System (20) eine Stereokamera (21, 22) mit einem entlang einer ersten optischen Achse (213) angeordneten ersten Stereoobjektiv (211) und einem entlang einer zweiten optischen Achse (223) angeordneten zweiten Stereoobjektiv (221) aufweist und der Stereowinkel α ein Winkel zwischen der ersten optischen Achse (213) und der zweiten optischen Achse (223) ist, und wobei die Steuereinheit (40) ferner dazu ausgebildet ist, den Stereowinkel α anhand einer Zoomstufe und/oder eines Arbeitsabstandes der Stereokamera (21, 22) zu ermitteln.

12. System (100) nach einem der vorangehenden Ansprüche, ferner aufweisend ein medizintechnisches Instrument (70), wobei die Steuereinheit (40) dazu eingerichtet ist, eine Position, einen Typ und/oder einen Zustand des medizintechnischen Instruments (70) zu ermitteln und die Phase der durchgeführten Operation anhand der Position, des Typs und/oder des Zustands des medizintechnischen Instruments (70) zu ermitteln.

13. Verfahren einer Steuereinheit eines Systems (100) zum Erfassen und Visualisieren von OCT-Signalen, das Verfahren aufweisend die Verfahrensschritte:
Erfassen (S100) eines zeitaufgelösten OCT-Signals (19) eines ausgewählten Sichtfelds (66) einer Probe (65) mittels eines OCT Systems (10), wobei das OCT-Signal (19) eine Vielzahl von Tupeln (191) aufweist, die jeweils ein Volumenelement (651) der Probe (65) und eine zu dem Volumenelement (651) korrespondierende Streuintensität repräsentieren;
Ermitteln einer Phase einer durchgeführten Operation;
Ermitteln (S200) einer vorgegebenen virtuellen Blickrichtung (60) und eines Stereowinkels α, wobei der Stereowinkel α und/oder die virtuelle Blickrichtung anhand der ermittelten Phase der durchgeführten Operation ermittelt werden;
Ermitteln (S300) eines zeitaufgelösten ersten OCT-Bilds (311) und eines zeitaufgelösten zweiten OCT-Bilds (312), jeweils anhand der Tupel (191), einer Auflösung eines Anzeigemittels (30),der virtuellen Blickrichtung (60) und des Stereowinkels α als dreidimensionales und aus der virtuellen Blickrichtung (60) perspektivisches Volumenbild; und
Stereoskopisches Darstellen (S400) des zeitaufgelösten ersten OCT-Bilds (311) und des zeitaufgelösten zweiten OCT-Bilds (312) auf dem Anzeigemittel (30).

14. Verfahren nach Anspruch 13, wobei das Ermitteln (S200) einer vorgegebenen virtuellen Blickrichtung (60) basierend auf einer mittels einer Schnittstelle (50) erfassten Nutzereingabe und/oder basierend auf einem mittels einer Geräteschnittstelle (55) erfassten Geräteparameter erfolgt.

15. Computerprogramm, umfassend Befehle, die bei der Ausführung durch eine Steuereinheit (40) eines Systems (100) gemäß einem der Ansprüche 1 bis 12 bewirken, dass das System (100) gemäß einem der Ansprüche 1 bis 12 ein Verfahren gemäß einem der Ansprüche 13 und 14 ausführt.

## Claims

1. System (100) for acquiring and visualizing OCT signals, comprising:
an OCT system (10);
a display means (30) designed for the time-resolved display of image data (31) as stereoscopic images; and
a control unit (40) configured to drive the OCT system (10) to acquire a time-resolved OCT signal (19) of a selected field of view (66) of the sample (65) and to determine a time-resolved first OCT image (311) and a time-resolved second OCT image (312) on the basis of the acquired time-resolved OCT signal (19), a specifiable virtual viewing direction (60) and a stereo angle α and to stereoscopically display the first time-resolved OCT image (311) and the second time-resolved OCT image (312) on the display means (30),
wherein the control unit (40) is further configured to determine a phase of a performed operation and to determine the stereo angle α and/or the virtual viewing direction on the basis of the determined phase of the performed operation.

2. System (100) according to Claim 1, wherein the virtual viewing direction (60) is defined by an azimuth angle (61) and an elevation angle (62) relative to the optical axis (63) of the OCT system (10).

3. System (100) according to Claim 1 or 2, wherein the control unit (40) is configured to determine the time-resolved OCT image (31) as a three-dimensional volume image from the perspective of the virtual viewing direction (60).

4. System according to any of the preceding claims, wherein the system (100) further comprises an interface (50) designed to acquire a user input and the virtual viewing direction is determined on the basis of a user input and/or wherein the system (100) further comprises a device interface (55) designed for acquiring a device parameter and the virtual viewing direction is determined on the basis of a device parameter acquired by means of the device interface (55).

5. System (100) according to any of the preceding claims, wherein the OCT signal (19) contains a multiplicity of tuples (191) each representing a volume element (651) of the sample (65) and a scattering intensity, wherein the display means (30) comprises a multiplicity of pixels (33) and wherein the control unit (40) is configured to determine OCT images (31) in such a way on the basis of the tuples (191), a resolution of the display means (30) and the virtual viewing direction (60) that certain pixels (33) correspond to certain volume elements (651).

6. System (100) according to any of the preceding claims, further comprising:
a surgical-microscopic system (20) designed to acquire a time-resolved image signal (23) of the selected field of view (66) of the sample (65),
wherein the control unit (40) is further configured to determine corresponding video image data (32) on the basis of the acquired time-resolved image signal (23) and to simultaneously or sequentially display the video image data (32) and the time-resolved OCT image (31) on the display means (30).

7. System (100) according to Claim 6, wherein the control unit (40) is configured to sequentially display the video image data (32) and the time-resolved OCT image (31) on the display means (30) with the same magnification, the same perspective and/or the same stereo angle α.

8. System (100) according to Claim 6, wherein the control unit (40) is configured to simultaneously display the video image data (32) with a first level of transparency and the time-resolved OCT image (31) with a second level of transparency on the display means (30).

9. System (100) according to Claim 1, wherein the control unit (40) is further configured to determine the stereo angle α on the basis of a user input acquired by means of the interface (50).

10. System (100) according to Claim 6, wherein the control unit (40) is further configured to determine the stereo angle α on the basis of optical parameters of the surgical-microscopic system (20).

11. System (100) according to Claim 6, wherein the surgical-microscopic system (20) comprises a stereo camera (21, 22) with a first stereo lens (211) arranged along a first optical axis (213) and a second stereo lens (221) arranged along a second optical axis (223) and the stereo angle α is an angle between the first optical axis (213) and the second optical axis (223), and wherein the control unit (40) is further designed to determine the stereo angle α on the basis of a zoom level and/or a working distance of the stereo camera (21, 22).

12. System (100) according to any of the preceding claims, further comprising a medical instrument (70), wherein the control unit (40) is configured to determine a position, a type and/or a state of the medical instrument (70) and to determine the phase of the performed operation on the basis of the position, the type and/or the state of the medical instrument (70).

13. Method of a control unit of a system (100) for acquiring and visualizing OCT signals, the method including the following method steps:
acquiring (S100) a time-resolved OCT signal (19) of a selected field of view (66) of a sample (65) by means of an OCT system (10), wherein the OCT signal (19) comprises a multiplicity of tuples (191) each representing a volume element (651) of the sample (65) and a scattering intensity corresponding to the volume element (651);
determining a phase of a performed operation;
determining (S200) a given virtual viewing direction (60) and a stereo angle α, wherein the stereo angle α and/or the virtual viewing direction are determined on the basis of the determined phase of the performed operation;
determining (S300) a time-resolved first OCT image (311) and a time-resolved second OCT image (312), in each case on the basis of the tuples (191), a resolution of a display means (30), the virtual viewing direction (60) and the stereo angle α, as a three-dimensional volume image from the perspective of the virtual viewing direction (60); and
stereoscopically displaying (S400) the time-resolved first OCT image (311) and the time-resolved second OCT image (312) on the display means (30).

14. Method according to Claim 13, wherein a given virtual viewing direction (60) is determined (S200) on the basis of a user input acquired by means of an interface (50) and/or on the basis of a device parameter acquired by means of a device interface (55).

15. Computer program comprising commands which, upon execution by a control unit (40) of a system (100) according to any of Claims 1 to 12, cause the system (100) according to any of Claims 1 to 12 to carry out a method according to either of Claims 13 and 14.

## Revendications

1. Système (100) d'acquisition et de visualisation de signaux OCT, comprenant :
un système OCT (10) ;
un moyen d'affichage (30) conçu pour l'affichage résolu dans le temps de données d'image (31) sous forme d'images stéréoscopiques ; et
une unité de commande (40), qui est conçue pour commander le système OCT (10) afin d'acquérir un signal OCT résolu dans le temps (19) d'un champ de vision sélectionné (66) de l'échantillon (65) et pour déterminer, sur la base du signal OCT résolu dans le temps (19) acquis, d'une direction de visualisation virtuelle prédéfinissable (60) et d'un angle stéréo α, une première image OCT résolue dans le temps (311) et une seconde image OCT résolue dans le temps (312), et pour afficher de manière stéréoscopique sur le moyen d'affichage (30) la première image OCT résolue dans le temps (311) et la seconde image OCT résolue dans le temps (312),
dans lequel l'unité de commande (40) est en outre conçue pour déterminer une phase d'une opération effectuée et pour déterminer l'angle stéréo α et/ou la direction de visualisation virtuelle sur la base de la phase déterminée de l'opération effectuée.

2. Système (100) selon la revendication 1, dans lequel la direction de visualisation virtuelle (60) est définie par un angle d'azimut (61) et un angle d'élévation (62) par rapport à l'axe optique (63) du système OCT (10).

3. Système (100) selon la revendication 1 ou 2, dans lequel l'unité de commande (40) est conçue pour déterminer l'image OCT résolue dans le temps (31), en tant qu'image volumique tridimensionnelle et en perspective suivant la direction de visualisation virtuelle (60).

4. Système selon l'une quelconque des revendications précédentes, dans lequel le système (100) comprend en outre une interface (50) conçue pour acquérir une entrée utilisateur, et la direction de visualisation virtuelle est déterminée sur la base d'une entrée utilisateur et/ou dans lequel le système (100) comporte en outre une interface d'appareil (55) conçue pour acquérir un paramètre de dispositif, et la direction de visualisation virtuelle est déterminée sur la base d'un paramètre d'appareil acquis au moyen de l'interface d'appareil (55).

5. Système (100) selon l'une quelconque des revendications précédentes, dans lequel le signal OCT (19) comporte une pluralité de tuples (191), qui représentent respectivement un élément de volume (651) de l'échantillon (65) et une intensité de diffusion, dans lequel le moyen d'affichage (30) comporte une pluralité de pixels (33) et dans lequel l'unité de commande (40) est conçue pour déterminer des images OCT (31) sur la base des tuples (191), d'une résolution du moyen d'affichage (30) et de la direction de visualisation virtuelle (60) de telle sorte que certains pixels (33) correspondent à certains éléments de volume (651).

6. Système (100) selon l'une quelconque des revendications précédentes, comportant en outre :
un système de microscope opératoire (20) conçu pour acquérir un signal d'image résolu dans le temps (23) du champ de vision sélectionné (66) de l'échantillon (65), dans lequel l'unité de commande (40) est en outre conçue pour déterminer des données d'image vidéo (32) correspondantes sur la base du signal d'image résolu dans le temps (23) acquis et pour afficher les données d'image vidéo (32) simultanément ou séquentiellement avec l'image OCT résolue dans le temps (31) sur le moyen d'affichage (30).

7. Système (100) selon la revendication 6, dans lequel l'unité de commande (40) est conçue pour afficher séquentiellement les données d'image vidéo (32) et l'image OCT résolue dans le temps (31) avec un même grossissement, une même perspective et/ou un même angle stéréo α sur le moyen d'affichage (30).

8. Système (100) selon la revendication 6, dans lequel l'unité de commande (40) est conçue pour afficher simultanément les données d'image vidéo (32) avec un premier niveau de transparence et l'image OCT résolue dans le temps (31) avec un second niveau de transparence sur le moyen d'affichage (30).

9. Système (100) selon la revendication 1, dans lequel l'unité de commande (40) est en outre conçue pour déterminer l'angle stéréo α sur la base d'une entrée utilisateur acquise au moyen de l'interface (50).

10. Système (100) selon la revendication 6, dans lequel l'unité de commande (40) est en outre conçue pour déterminer l'angle stéréo α sur la base de paramètres optiques du système de microscope opératoire (20).

11. Système (100) selon la revendication 6, dans lequel le système de microscope opératoire (20) comporte une caméra stéréo (21, 22) comprenant un premier objectif stéréo (211) disposé le long d'un premier axe optique (213) et un second objectif stéréo (221) disposé le long d'un second axe optique (223), et l'angle stéréo α est un angle formé entre le premier axe optique (213) et le second axe optique (223), et dans lequel l'unité de commande (40) est en outre conçue pour déterminer l'angle stéréo α sur la base d'un niveau de zoom et/ou d'une distance de travail de la caméra stéréo (21, 22).

12. Système (100) selon l'une quelconque des revendications précédentes, comportant en outre un instrument médical (70), dans lequel l'unité de commande (40) est conçue pour déterminer une position, un type et/ou un état de l'instrument médical (70) et pour déterminer la phase de l'opération effectuée sur la base de la position, du type et/ou de l'état de l'instrument médical (70).

13. Procédé d'une unité de commande d'un système (100) d'acquisition et de visualisation de signaux OCT, le procédé comportant les étapes de procédé suivantes :
acquisition (S100) d'un signal OCT résolu dans le temps (19) d'un champ de vision sélectionné (66) d'un échantillon (65) au moyen d'un système OCT (10), le signal OCT (19) comportant une pluralité de tuples (191), qui représentent respectivement un élément de volume (651) de l'échantillon (65) et une intensité de diffusion correspondant à l'élément de volume (651) ;
détermination d'une phase d'une opération effectuée ;
détermination (S200) d'une direction de visualisation virtuelle prédéfinie (60) et d'un angle stéréo α, l'angle stéréo α et/ou la direction de visualisation virtuelle étant déterminés sur la base de la phase déterminée de l'opération effectuée ;
détermination (S300) d'une première image OCT résolue dans le temps (311) et d'une seconde image OCT résolue dans le temps (312), respectivement sur la base des tuples (191), d'une résolution d'un moyen d'affichage (30), de la direction de visualisation virtuelle (60) et de l'angle stéréo α, en tant qu'image volumique tridimensionnelle et en perspective suivant la direction de visualisation virtuelle (60) ; et
affichage stéréoscopique (S400) sur le moyen d'affichage (30) de la première image OCT résolue dans le temps (311) et de la seconde image OCT résolue dans le temps (312).

14. Procédé selon la revendication 13, dans lequel la détermination (S200) d'une direction de visualisation virtuelle prédéfinie (60) est effectuée sur la base d'une entrée utilisateur acquise au moyen d'une interface (50) et/ou sur la base d'un paramètre d'appareil acquis au moyen d'une interface d'appareil (55).

15. Programme informatique comprenant des instructions qui, lorsqu'elles sont exécutées par une unité de commande (40) d'un système (100) selon l'une quelconque des revendications 1 à 12, amènent le système (100) selon l'une quelconque des revendications 1 à 12 à mettre en œuvre un procédé selon l'une quelconque des revendications 13 et 14.
